# EUROPEAN PATENT APPLICATION

(11) **EP 4 792 834 A1**
(43) Date of publication of application: **19.08.2026**
(21) Application number: 24876626.3
(22) Date of filing: 11.10.2024
(51) Int. Cl.: A61K 47/68, A61P 35/00, C07K 16/28, A61K 31/4745

(54) **METHOD OF USING CLAUDIN18.2-TARGETED ANTIBODY-DRUG CONJUGATE IN COMBINATION THERAPY FOR CANCER**

(30) Priority: 11.10.2023 CN 202311309831
(71) Applicant: Fortvita Biologics (Singapore) Pte. Ltd., Singapore 189767 (SG)
(72) Inventor: ZHOU, Hui, Suzhou, Jiangsu 215123 (CN); LUO, Yang, Suzhou, Jiangsu 215123 (CN); ZHAO, Xin, Suzhou, Jiangsu 215123 (CN)
(74) Representative: Heath, Abigail
(86) International application number: PCT/CN2024/124096
(87) International publication number: WO 2025/077815

(57) **Abstract**

The present invention relates to a method of using a Claudin1 8.2-targeted antibody-drug conjugate in combination therapy for cancer. The Claudin18.2-targeted antibody-drug conjugate of the present invention, when used in combination with antibody drugs for treating cancer and/or chemotherapy drugs, has good therapeutic effects in 5 treating various types of tumors and subjects having different CLDN18.2 expression levels.

## Description

### TECHNICAL FIELD

The present invention relates to the field of disease treatment. In particular, the present invention relates to a method for treating cancer with a Claudin18.2-targeted antibody-drug conjugate.

### BACKGROUND ART

Gastric cancer is one of the most common malignant tumors in the world and one of the leading causes of cancer-related death worldwide. According to data from GLOBOCAN, the incidence and mortality of gastric cancer ranked fifth and fourth in 2020, respectively, and the 5-year survival rate of patients with metastatic gastric cancer was less than 5%. At present, surgical resection remains the only radical treatment for gastric cancer, and chemotherapy with a combination of fluoropyrimidine and platinum is the standard treatment for patients with advanced metastatic gastric cancer. With the advent of antiangiogenic and targeted therapies such as trastuzumab and ramucirumab, treatment options for gastric cancer have increased. However, systemic treatment has limited efficacy in advanced gastric cancer, with a median survival of only about 1 year. In terms of immunotherapy, nivolumab was approved in Japan for the treatment of patients with advanced gastric and gastroesophageal junction cancer after failure of at least two systemic therapies based on the results of ATTRACTION-02 study. In April 2021, based on the results of CheckMate-649 study, the FDA approved nivolumab in combination with fluoropyrimidine- and platinum-based chemotherapy as the first-line therapy for advanced or metastatic gastric cancer, gastroesophageal junction cancer, and esophageal adenocarcinoma, which was also the first FDA-approved first-line immunotherapy for gastric cancer. Despite these encouraging results, new therapies are needed to benefit more gastric cancer patients.

Pancreatic cancer is one of the most common malignant digestive system tumors. In 2020, there were 496,000 new cases of pancreatic cancer and 466,000 deaths worldwide, ranking seventh among all malignant tumors. Pancreatic cancer has a poor prognosis because it is often diagnosed at an advanced stage. According to statistics from the Surveillance, Epidemiology and End Results (SEER) database of the National Cancer Institute, the 5-year survival rate for pancreatic cancer is only 8%, and only 28.6% of patients survive more than 1 year after initial diagnosis. In addition, due to the high degree of malignancy, recurrence is common in patients with pancreatic cancer despite surgical intervention, and the 5-year survival rate of patients with pancreatic cancer after radical resection is only 18-27%. Pancreatic ductal adenocarcinoma (PDAC) is the most common type of pancreatic cancer, accounting for more than 90% of pancreatic cancer cases. Despite the application of traditional therapies such as chemotherapy, surgery, and radiation therapy, no significant improvement in survival has been found. Surgical resection and chemotherapy (gemcitabine in combination with nab-paclitaxel and FOLFIRINOX) have been shown to improve survival in patients with early-stage pancreatic cancer, but these are insufficient for the treatment of patients with advanced pancreatic cancer.

Some drugs targeting CLDN18.2 have entered clinical studies and showed good efficacy and safety. Zolbetuximab (IMAB362, claudixmab, Astellas) is the first drug developed for the CLDN18.2 target. As a chimeric IgG1 monoclonal antibody, it can specifically bind to CLDN18.2 on the surface of tumor cells, causing antibody-dependent cell-mediated cytotoxicity (ADCC), complement dependent cytotoxicity (CDC), apoptosis, and cell proliferation inhibition. Subsequently, several clinical trials have also evaluated its efficacy and safety in patients with gastroesophageal cancer. A phase IIa trial (NCT01197885, MONO) assessed the efficacy and safety of zolbetuximab monotherapy in 54 patients with relapsed or refractory advanced gastric or lower esophageal adenocarcinoma, with moderate or high CLDN18.2 expression in ≥ 50% of tumor cells. The study showed that 10 of the 43 evaluable subjects had clinical benefits, of which 4 achieved partial response (PR) (objective response rate (ORR) 9%) and 6 (14%) achieved stable disease (SD). The median duration of response (DoR) for subjects who responded to zolbetuximab was 24.6 weeks (range: 13.1-156.1 weeks). Among the 10 subjects who obtained clinical benefit (complete response (CR) + PR + SD), 9 (90%) had moderate or high CLDN18.2 expression in ≥ 70% of tumor cells, and subgroup analysis of these 9 subjects found that 4 (14%) achieved PR and 5 (17%) achieved SD. Among all subjects, 52 (96%) experienced treatment-emergent adverse events (TEAEs), most of which were Grade 1 or 2. TEAEs occurring in ≥30% of subjects included nausea (63%), vomiting (57%), fatigue (43%), and decreased appetite (30%). Grade 3 vomiting was reported in 12 subjects (22%) and Grade 3 nausea was reported in 8 subjects (15%). Two subjects had Grade 4 TEAEs: dyspnea and diarrhea (1 subject each). 44 subjects (82%) experienced treatment-related adverse events (TRAEs), of which nausea, vomiting, fatigue, and decreased appetite occurred in ≥ 10% of subjects. Only 5 subjects experienced serious TRAEs. Both Grade 3 and Grade 2 TEAEs (nausea and vomiting) occurred in 1 subject, Grade 2 hematemesis occurred in 1 subject and Grade 3 vomiting occurred in 2 subjects. All serious TRAEs occurred in subjects who received the 600 mg/m² dose, except for 1 subject who experienced Grade 3 vomiting. These TEAEs can be alleviated by pausing or slowing down the infusion of zolbetuximab. Patients who do not have prior gastrectomy are more likely to report nausea and vomiting. The incidence of these TEAEs decreased over time as subjects received repeated zolbetuximab infusions.

In another phase II trial (NCT01630083, FAST) evaluating the efficacy and safety of zolbetuximab combined with EOX vs. EOX in the first-line treatment of CLDN18.2-positive advanced gastric/gastroesophageal junction adenocarcinoma (G/GEJ AC), the results showed that the median progression-free survival (PFS) was improved from 5.3 months to 7.5 months [hazard ratio (HR), 0.44; 95% CI, 0.29-0.67; P < 0.0005]; the median overall survival (OS) was increased from 8.3 months to 13.0 months (HR, 0.55; 95% CI, 0.39-0.77; P < 0.0005). The most frequently reported TEAEs in the zolbetuximab combined with EOX group were nausea (81.8%) and vomiting (67.5%), with a low incidence of vomiting in patients experiencing previous gastrectomy, and there was a dose-dependent relationship between the incidence and severity of vomiting and zolbetuximab. The most commonly reported serious adverse events (SAEs) in the zolbetuximab combined with EOX group were malignancy (3.9%), pneumonia (2.6%), and febrile neutropenia (2.6%), and the most commonly reported SAEs in the EOX group were malignancy (8.3%), gastrorrhagia (3.6%) and nausea, renal failure, pulmonary embolism, and febrile neutropenia (2.4% each). Two phase III clinical studies (NCT03504397/NCT03653507) evaluating the efficacy of zolbetuximab combined with different chemotherapy regimens (mFOLFOX6 or CAPOX) vs. placebo combined with chemotherapy as first-line treatment for CLDN18.2-positive and HER2-negative locally advanced unresectable or metastatic G/GEJ AC are ongoing. These data provide safety and efficacy data for drugs targeting CLDN18.2.

In addition to the CLDN18.2-targeted monoclonal antibody, CLDN18.2-targeted ADC drugs are also being developed one after another. At present, the ADC drugs CMG901 and SYSA1801 targeting CLDN18.2 that have progressed rapidly have both shown good safety and tolerability and preliminary efficacy in treating CLDN18.2-positive drug-resistant/refractory solid tumors in their phase I studies.

There is increasing evidence that ADCs may enhance the efficacy of immunotherapeutic agents. The mechanisms involved are diverse, including induction of immunogenic cell death, maturation of dendritic cells, increased T-lymphocyte infiltration, and enhancement of immune memory and expression of immunomodulatory proteins (such as PD-L1 and MHC). Some ADCs exhibit greater potency in preclinical models with intact immune systems, supporting the relevance of their immunomodulatory function. A number of HER2-targeted ADCs, including trastuzumab emtansine, trastuzumab deruxtecan and disitamab vedotin, have been tested in combination with ICIs in vitro and in vivo, demonstrating synergistic activity associated with enhanced homing and immune effector activation.

ADCs have received regulatory approval as monotherapy for a variety of solid and hematologic malignancies and have shown anti-tumor efficacy. ADC in combination with immunotherapy, chemotherapy, or anti-angiogenic therapy has shown better anti-tumor effects than monotherapy in both preclinical and clinical studies, and a combination therapy with efficacy and safety recognized by regulatory authorities has obtained accelerated approval from the FDA. However, no relevant exploration results have been disclosed in subjects with different tumor types positive for CLDN18.2 expression.

### SUMMARY OF THE INVENTION

In order to solve the above technical problems, an objective of the present invention is to provide a method for treating cancer using a Claudin18.2-targeted antibody-drug conjugate.

In order to achieve the above objective, the present invention provides the following aspects:
A unit formulation, wherein the unit formulation comprises: an effective amount (e.g., 1 mg -10000 mg, 5 mg-9000 mg, 3 mg-8000 mg, 10 mg-6000 mg, 50 mg-4000 mg, 100 mg-2500 mg, 200 mg-1000 mg, 300 mg-900 mg, 400 mg-800 mg, 500 mg-700 mg, 180 mg-550 mg, 200 mg-540 mg, 240 mg-480 mg, 300 mg-420 mg, 360 mg-420 mg, or 360 mg-480 mg) of a Claudin18.2-targeted antibody-drug conjugate.

A single drug dose unit or kit, comprising an effective amount (e.g., 1 mg-10000 mg, 5 mg-9000 mg, 3 mg-8000 mg, 10 mg-6000 mg, 50 mg-4000 mg, 100 mg-2500 mg, 200 mg-1000 mg, 300 mg-900 mg, 400 mg-800 mg, 500 mg-700 mg, 180 mg-550 mg, 200 mg-540 mg, 240 mg-480 mg, 300 mg-420 mg, 360 mg-420 mg, or 360 mg-480 mg) of a Claudin18.2-targeted antibody-drug conjugate, preferably further comprising an effective amount (e.g., 1 mg-10000 mg, 5 mg-9000 mg, 3 mg-8000 mg, 10 mg-6000 mg, 50 mg-4000 mg, 100 mg-2500 mg, 200 mg-1000 mg, 300 mg-900 mg, 400 mg-800 mg, 500 mg-700 mg, 180 mg-550 mg, 200 mg-540 mg, 240 mg-480 mg, 300 mg-420 mg, 360 mg-420 mg, or 360 mg-480 mg) of an antibody drug for treating cancer, and/or a chemotherapy drug.

Use of a Claudin18.2-targeted antibody-drug conjugate in preparing a pharmaceutical composition or kit for treating cancer, wherein the pharmaceutical composition or kit comprises an effective amount of the Claudin18.2-targeted antibody-drug conjugate and a component A selected from an antibody drug for treating cancer and/or a chemotherapy drug.

In one or more embodiments of the present invention, the Claudin18.2-targeted antibody-drug conjugate, the antibody drug for treating cancer and the chemotherapy drug are in a form suitable for injection (preferably intravenous or subcutaneous injection) or oral administration.

In one or more embodiments of the present invention, the unit formulation, the single drug dose unit, the pharmaceutical composition, and the kit further comprise instructions.

In one or more embodiments of the present invention, the specification describes a drug administration regimen, for example, the Claudin18.2-targeted antibody-drug conjugate is administered at a dose of 0.1 mg/kg-100 mg/kg, 0.2 mg/kg-50 mg/kg, 0.3 mg/kg-30 mg/kg, 0.3 mg/kg, 1 mg/kg, 3 mg/kg, 4.5 mg/kg, 6 mg/kg, 7.5 mg/kg, 8 mg/kg, 10 mg/kg, 11 mg/kg, 12 mg/kg, 13.5 mg/kg, 15 mg/kg, 16 mg/kg, 18 mg/kg, 20 mg/kg, 22 mg/kg, 25 mg/kg, 28 mg/kg, or 30 mg/kg, with an administration cycle of 2 weeks/14 days (Q2W) to 5 weeks/35 days (Q5W), preferably an administration cycle of 3 weeks/21 days (Q3W) or 4 weeks/28 days (Q4W), and administered once on day 1 of each cycle.

In one or more embodiments of the present invention, the effective amount of the Claudin18.2-targeted antibody-drug conjugate is selected from the group consisting of: 0.1 mg/kg-100 mg/kg, 0.2 mg/kg-50 mg/kg, 0.3 mg/kg-30 mg/kg, 1 mg/kg, 3 mg/kg, 4.5 mg/kg, 6 mg/kg, 7.5 mg/kg, 8 mg/kg, 10 mg/kg, 12 mg/kg, 13.5 mg/kg, 15 mg/kg, 16 mg/kg, 18 mg/kg, 20 mg/kg, 22 mg/kg, 25 mg/kg, 28 mg/kg, or 30 mg/kg.

In one or more embodiments of the present invention, the effective amount of the Claudin18.2-targeted antibody-drug conjugate is selected from the group consisting of: 1 mg-10000 mg, 5 mg-9000 mg, 3 mg-8000 mg, 10 mg-6000 mg, 50 mg-4000 mg, 100 mg-2500 mg, 200 mg-1000 mg, 300 mg-900 mg, 400 mg-800 mg, 500 mg-700 mg, 180 mg-550 mg, 200 mg-540 mg, 240 mg-480 mg, 300 mg-420 mg, 360 mg-420 mg, or 360 mg-480 mg.

In order to achieve the above objective, the present invention further provides a method for treating cancer with a Claudin18.2-targeted antibody-drug conjugate, comprising administering to an individual in need thereof an effective amount of the Claudin18.2-targeted antibody-drug conjugate and an antibody drug for treating cancer and/or a chemotherapy drug, wherein preferably, the administration mode is injection (preferably intravenous injection or subcutaneous injection) or oral administration.

In one or more embodiments of the present invention, the Claudin18.2-targeted antibody-drug conjugate is selected from: 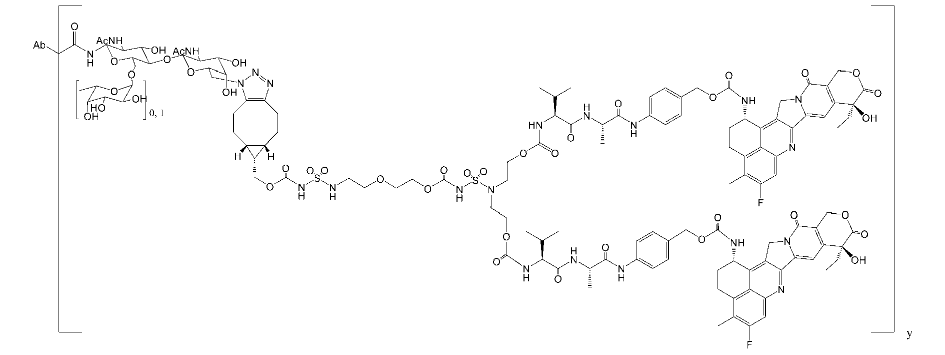
wherein Ab is a Claudin18.2-targeted antibody or an antigen-binding fragment thereof;
wherein y is an integer of 1 or 2; or, 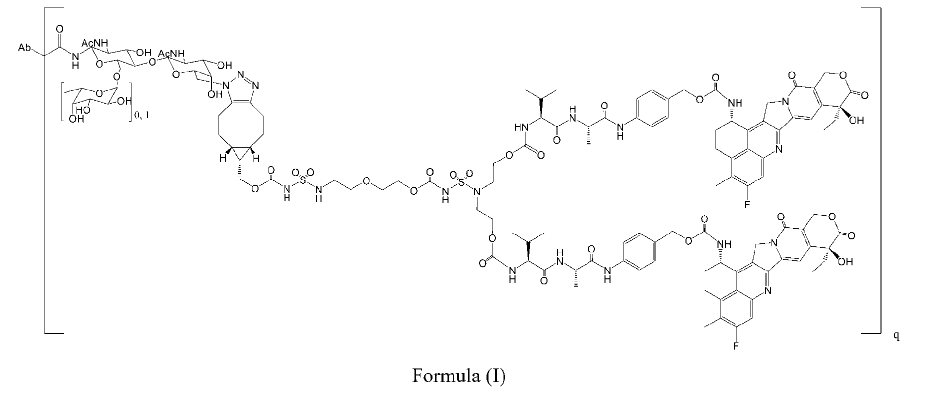
wherein Ab is a Claudin18.2-targeted antibody or an antigen-binding fragment thereof;
wherein q represents an average DAR, and q is 2-11.
wherein the Claudin18.2-targeted antibody or the antigen-binding fragment thereof comprises HCDR1, HCDR2 and HCDR3 set forth in SEQ ID NO: 1 (GFTFSSYVMS), SEQ ID NO: 2 (TISHSGGSTYYADSVKG) and SEQ ID NO: 3 (DAPYYDILTGYRY), respectively, and LCDR1, LCDR2 and LCDR3 set forth in SEQ ID NO: 6 (RASQSISSWLA), SEQ ID NO: 7 (KASSLES) and SEQ ID NO: 8 (QQYNSYSYT), respectively.

In one or more embodiments of the present invention, the Claudin18.2-targeted antibody or the antigen-binding fragment thereof comprises a heavy chain variable region and/or a light chain variable region, wherein the heavy chain variable region
(i) comprises or consists of an amino acid sequence set forth in SEQ ID NO: 4; or
(ii) comprises or consists of the following sequences: an amino acid sequence having one or more (preferably no more than 10, and more preferably no more than 5, 4, 3, 2, or 1) amino acid changes (preferably amino acid substitutions, and more preferably conservative amino acid substitutions) compared to an amino acid sequence set forth in SEQ ID NO: 4 (preferably, the amino acid changes do not occur in the CDR regions); or

an amino acid sequence having at least 85%, at least 90%, at least 91%, at least 92%, at least 93%, at least 94%, at least 95%, at least 96%, at least 97%, at least 98%, or at least 99% sequence identity to the amino acid sequence of SEQ ID NO: 4; and/or
the light chain variable region
   (i) comprises or consists of an amino acid sequence set forth in SEQ ID NO: 9; or
   (ii) comprises or consists of the following sequence: an amino acid sequence having one or more (preferably no more than 10, and more preferably no more than 5, 4, 3, 2, or 1) amino acid changes (preferably amino acid substitutions, and more preferably conservative amino acid substitutions) compared to an amino acid sequence set forth in SEQ ID NO: 9 (preferably, the amino acid changes do not occur in the CDR regions); or
an amino acid sequence having at least 85%, at least 90%, at least 91%, at least 92%, at least 93%, at least 94%, at least 95%, at least 96%, at least 97%, at least 98%, or at least 99% sequence identity to the amino acid sequence of SEQ ID NO: 9.

In one or more embodiments of the present invention, the Claudin18.2-targeted antibody further comprises a heavy chain constant region and/or a light chain constant region. In one or more embodiments of the present invention, the heavy chain constant region of the Claudin18.2-targeted antibody or is an IgG antibody.

In one or more embodiments of the present invention, the heavy chain constant region of the Claudin18.2-targeted antibody is selected from IgG1, IgG2, IgG3, or IgG4, preferably IgG1, more preferably human IgG1, e.g., a (human) wild-type IgG1 heavy chain constant region.

In one or more embodiments, the light chain constant region (LC) of the antibody of the present invention is a lambda or Kappa light chain constant region.

In one or more embodiments, the heavy chain constant region (HC) of the present invention
(i) comprises or consists of an amino acid sequence having at least 85%, at least 90%, at least 91%, at least 92%, at least 93%, at least 94%, at least 95%, at least 96%, at least 97%, at least 98%, or at least 99% identity to an amino acid sequence set forth in SEQ ID NO: 5;
(ii) comprises or consists of an amino acid sequence set forth in SEQ ID NO: 5; or
(iii) comprises or consists of an amino acid sequence having one or more (preferably no more than 20 or 10, and more preferably no more than 5, 4, 3, 2, or 1) amino acid changes (preferably amino acid substitutions, and more preferably conservative amino acid substitutions) compared to the amino acid sequence set forth in SEQ ID NO: 5.

In some preferred embodiments, the light chain constant region (LC) of the antibody of the present invention
(i) comprises or consists of an amino acid sequence having at least 85%, at least 90%, at least 91%, at least 92%, at least 93%, at least 94%, at least 95%, at least 96%, at least 97%, at least 98%, or at least 99% identity to an amino acid sequence set forth in SEQ ID NO: 10;
(ii) comprises or consists of an amino acid sequence set forth in SEQ ID NO: 10; or
(iii) comprises or consists of an amino acid sequence having one or more (preferably no more than 20, and more preferably no more than 5, 4, 3, 2, or 1) amino acid changes (preferably amino acid substitutions, and more preferably conservative amino acid substitutions) compared to the amino acid sequence set forth in SEQ ID NO: 10.

In one or more embodiments of the present invention, the Claudin18.2-targeted antibody comprises a heavy chain and a light chain, wherein the heavy chain
comprises or consists of an amino acid sequence set forth in SEQ ID NO: 11; or
comprises or consists of the following sequence: an amino acid sequence having one or more (preferably no more than 20 or 10, and more preferably no more than 5, 4, 3, 2, or 1) amino acid changes (preferably amino acid substitutions, and more preferably conservative amino acid substitutions) compared to the amino acid sequence set forth in SEQ ID NO: 11, or an amino acid sequence having at least 85%, at least 90%, at least 91%, at least 92%, at least 93%, at least 94%, at least 95%, at least 96%, at least 97%, at least 98%, or at least 99% sequence identity to the amino acid sequence set forth in SEQ ID NO: 11; and
the light chain
comprises or consists of an amino acid sequence set forth in SEQ ID NO: 12; or
comprises or consists of the following sequence: an amino acid sequence having one or more (preferably no more than 20 or 10, and more preferably no more than 5, 4, 3, 2, or 1) amino acid changes (preferably amino acid substitutions, and more preferably conservative amino acid substitutions) compared to an amino acid sequence set forth in SEQ ID NO: 12, or an amino acid sequence having at least 85%, at least 90%, at least 91%, at least 92%, at least 93%, at least 94%, at least 95%, at least 96%, at least 97%, at least 98%, or at least 99% sequence identity to the amino acid sequence set forth in SEQ ID NO: 12.

In one or more embodiments of the present invention, the method comprises administering to an individual in need thereof a Claudin18.2-targeted antibody-drug conjugate at 0.1 mg/kg-100 mg/kg.

In one or more embodiments of the present invention, the method comprises administering to an individual in need thereof a Claudin18.2-targeted antibody-drug conjugate at 0.2 mg/kg-50 mg/kg.

In one or more embodiments of the present invention, the method comprises administering to an individual in need thereof a Claudin18.2-targeted antibody-drug conjugate at 0.3 mg/kg-30 mg/kg.

In one or more embodiments of the present invention, the method comprises administering to an individual in need thereof a Claudin18.2-targeted antibody-drug conjugate at 1 mg/kg, 3 mg/kg, 4.5 mg/kg, 6 mg/kg, 7.5 mg/kg, 8 mg/kg, 10 mg/kg, 12 mg/kg, 13.5 mg/kg, 15 mg/kg, 16 mg/kg, 18 mg/kg, 20 mg/kg, 22 mg/kg, 25 mg/kg, 28 mg/kg, or 30 mg/kg.

In one or more embodiments of the present invention, the administration cycle of the Claudin18.2-targeted antibody-drug conjugate is 2 weeks/14 days (Q2W) - 5 weeks/35 days (Q5W). Further, the administration cycle is 2 weeks/14 days (Q2W), 3 weeks/21 days (Q3W), 4 weeks/28 days (Q4W), or 5 weeks/35 days (Q5W), and the administration is performed once on day 1 of each cycle.

In one or more embodiments of the present invention, the administration cycle of the Claudin18.2-targeted antibody-drug conjugate is 3 weeks/21 days (Q3W) or 4 weeks/28 days (Q4W), and the administration is performed once on day 1 of each cycle.

In one or more aspects of the present invention, the antibody drug for treating cancer is selected from one or a combination of more of a PD-1 inhibitor, a recombinant anti-VEGF monoclonal antibody, and a monoclonal antibody that specifically binds to VEGFR-2.

In one or more embodiments of the present invention, the PD-1 inhibitor is selected from one or a combination of more of pembrolizumab, nivolumab, camrelizumab, sintilimab, toripalimab, and tislelizumab.

In one or more embodiments of the present invention, the administration regimen of the PD-1 inhibitor is the administration regimen in the respective instructions of the PD-1 inhibitor.

In one or more embodiments of the present invention, the dosage of sintilimab is 200 mg, and preferably the administration cycle of sintilimab is 3 weeks/21 days (Q3W), e.g., administration on day 1.

In one or more embodiments of the present invention, the recombinant anti-VEGF monoclonal antibody is selected from one or a combination of two of sevacizumab and bevacizumab.

In one or more embodiments of the present invention, the dosage of bevacizumab is 15 mg/kg, and preferably, the administration cycle of bevacizumab is 3 weeks/21 days (Q3W), e.g., administration on day 1.

In one or more embodiments of the present invention, the monoclonal antibody that specifically binds to VEGFR-2 is ramucirumab.

In one or more embodiments of the present invention, the dosage of ramucirumab is 8 mg/kg, and preferably, the administration cycle of ramucirumab is 4 weeks/28 days (Q4W), e.g., administration on day 1 and day 15.

In one or more aspects of the present invention, the chemotherapy drug is selected from one or a combination of more of oxaliplatin, capecitabine, gemcitabine, albumin-bound paclitaxel, and tegafur, gimeracil and oteracil potassium.

In one or more embodiments of the present invention, the administration dose of oxaliplatin is 130 mg/m²; preferably, the administration cycle of oxaliplatin is 3 weeks/21 days (Q3W), e.g., administration on day 1.

In one or more embodiments of the present invention, the administration dose of capecitabine is 1000 mg/m²; preferably, the administration cycle of capecitabine is twice daily (BID).

In one or more embodiments of the present invention, the administration dose of gemcitabine is 1000 mg/m²; preferably, the administration cycle of gemcitabine is 3 weeks/21 days (Q3W), e.g., administration on day 1 and day 8.

In one or more embodiments of the present invention, the administration dose of the albumin-bound paclitaxel is 125 mg/m²; preferably, the administration cycle of the albumin-bound paclitaxel is 3 weeks/21 days (Q3W), e.g., administration on day 1 and day 8.

In one or more embodiments of the present invention, the administration dose of the tegafur, gimeracil and oteracil potassium is 40-60 mg; preferably, the administration cycle of the tegafur, gimeracil and oteracil potassium is 3 weeks/21 days (Q3W), twice daily (BID), e.g., administration on days 1-14.

In one or more embodiments of the present invention, the cancer comprises a solid tumor, and the solid tumor comprises gastric/gastroesophageal junction adenocarcinoma, pancreatic ductal adenocarcinoma.

In one or more embodiments of the present invention, the administration mode of the method comprises intravenous injection and oral administration.

The Claudin18.2-targeted antibody-drug conjugate of the present invention, as an ADC with a bystander effect, also has a significant tumor inhibitory effect in a mouse tumor-bearing model with low CLDN18.2 expression. In preclinical pharmacodynamic studies, it shows a certain synergistic effect whether used in combination with an antibody drug for treating cancer and/or a chemotherapy drug. Therefore, the Claudin18.2-targeted antibody-drug conjugate of the present invention in combination with an antibody drug for treating cancer and/or a chemotherapy drug has certain efficacy in treating subjects with various tumor types and different CLDN18.2 expression levels, and can significantly improve PFS and OS.

Other embodiments of the present invention will become apparent from the following detailed description.

### DETAILED DESCRIPTION OF THE INVENTION

Before describing the present invention in detail, it is to be understood that this invention is not limited to the particular methods and experimental conditions described herein, as such methods and conditions may vary. Additionally, the terminology used herein is for the purpose of describing particular embodiments only and is not intended to be limiting.

### DEFINITIONS

Unless defined otherwise, all technical and scientific terms used herein have the same meaning as commonly understood by one of ordinary skill in the art. For the purposes of the present invention, the following terms are defined below.

The term "about", when used in conjunction with a number, is meant to encompass numerical values within a range having a lower limit less than the specified number by 5% and an upper limit greater than the specified number by 5%.

The term "and/or", when used to connect two or more options, should be understood to refer to any one of the options or any two or more of the options.

As used herein, the term "comprise" or "include" is intended to mean that the described elements, integers or steps are included, but not to exclude any other elements, integers or steps. As used herein, the term "comprise" or "include", unless otherwise specified, also encompasses the situation where the entirety consists of the described elements, integers or steps. For example, when referring to an antibody variable region "comprising" a particular sequence, it is also intended to encompass an antibody variable region consisting of that particular sequence.

As used herein, the term "CLAUDIN" or "Claudin" or "CLDN" is the most important scaffold protein that determines the structure of tight junctions between cells, is involved in adhesion junctions, and plays an important role in metastasis and invasion of tumor cells. Claudin proteins are widely present in mammalian epithelial and endothelial cells, and are mainly distributed on lateral surface of the epithelial cell and on the basal cell plasma membrane. Different Claudin proteins are specifically expressed in different tissues, wherein the Claudin18 (CLDN18) gene is located at 3q22.3, has a molecular weight of 24 kDa, contains 261 amino acid residues, is a member of the Claudins superfamily, and has a protein structure comprising 2 extracellular loops and 4 transmembrane regions. The two subtypes of human CLDN18 or Claudin18 protein are Claudin18.1 or CLDN18.1 (UniProt ID: P56856-1) and Claudin18.2 or CLDN18.2 (UniProt ID: P56856-2), respectively. In the primary structural sequences of the two proteins, only the amino acid residues at certain positions from the N-terminal signal peptide to the extracellular loop 1 (Loop1) structure are different, especially on the extracellular loop 1. CLDN18.1 and CLDN18.2 differ in only 8 amino acids. The interspecies sequence homology of the two sub- proteins of CLDN18 is also very high, wherein the sequences of the extracellular loop 1 of CLDN18.2 are completely identical in different species such as human, mouse, and rhesus monkey, while the homology of the CLDN18.2 proteins of human and mouse reaches 84%, indicating that the sequence of the CLDN18.2 protein is extremely conserved (O. Tureci. et al., Gene 481:83-92, 2011). CLDN18.2, or any variants and isoforms thereof, can be isolated from cells or tissues in which they are naturally expressed, or recombinantly produced using techniques well known in the art and/or those described herein. In one embodiment, the CLDN18.2 described herein is human CLDN18.2.

The term "CLDN18.2-targeted antibody", "anti-CLDN18.2 antibody", "anti-CLDN18.2", "CLDN18.2 antibody" or "antibody binding to CLDN18.2" or "antibody specifically binding to CLDN18.2" as used herein refers to an antibody that is capable of binding to (human) CLDN18.2 with sufficient affinity such that the antibody can be used as a therapeutic agent targeting (human) CLDN18.2. In one embodiment, the (human) CLDN18.2 antibody binds to (human) CLDN18.2 with high affinity in vitro or in vivo. In one embodiment, the (human) CLDN18.2 antibody does not bind to CLDN18.1. In one embodiment, the (human) CLDN18.2 antibody binds to a cell expressing CLDN18.2 but does not bind to a cell expressing CLDN18.1. In some embodiments, the binding is measured, for example, by radioimmunoassay (RIA), bio-layer interferometry (BLI), MSD electrochemiluminescence detection technology assay, surface plasmon resonance (SPR), or flow cytometry.

The expression of CLDN18.2 in cells can be determined by a variety of means, e.g., an anti-CLDN18.2 antibody. For example, the binding strength of a cell "highly expressing CLDN18.2" to an anti-CLDN18.2 antibody (e.g., as determined by FACS) may be 500 times, 600 times, 700 times, 800 times, 900 times, or preferably 1000 times or more, e.g., 1100 times, 1200 times, 1300 times, or 1400 times or more, or more, the binding strength of the anti-CLDN18.2 antibody to a cell not expressing CLDN18.2. For example, the binding strength of a cell "moderately expressing CLDN18.2" to an anti-CLDN18.2 antibody (e.g., as determined by FACS) may be 5-500 times, e.g., 6, 7, 8, 9, 10, 15, 20, 30, 40, 50, 60, 70, 80, 90, 100 times or more, but no more than 500 times, the binding strength of the anti-CLDN18.2 antibody to a cell not expressing CLDN18.2.

For a conventional four-chain IgG antibody, a heavy chain of the full-length antibody generally consists of a heavy chain variable region (abbreviated herein as VH) and a heavy chain constant region, wherein the heavy chain constant region comprises at least 3 domains, CH1, CH2 and CH3. A light chain of the full-length antibody consists of a light chain variable region (abbreviated herein as VL) and a light chain constant region, wherein the light chain constant region consists of one domain CL. Each heavy chain variable region VH and each light chain variable region consists of three CDRs and four FRs arranged from the amino-terminus to the carboxyl-terminus in the following order: FR1, CDR1, FR2, CDR2, FR3, CDR3 and FR4.

The term "antibody fragment" includes a portion of an intact antibody. In a preferred embodiment, the antibody fragment is an antigen-binding fragment.

An "antigen-binding fragment" refers to a molecule other than an intact antibody that comprises a portion of an intact antibody and binds to an antigen to which the intact antibody binds. Examples of the antigen-binding fragment include, but are not limited to, Fv, Fab, Fab', Fab'-SH, F(ab')₂, a domain antibody (dAb), a linear antibody, a single-chain antibody (e.g., scFv), a single-domain antibody (e.g., VHH), a bivalent antibody or a fragment thereof, or a camelid antibody.

The term "antigen" refers to a molecule that elicits an immune response. This immune response may involve antibody production or activation of specific immune cells, or both. The skilled person will appreciate that any macromolecule, including substantially all proteins or peptides, may be used as an antigen. In addition, antigens may be derived from recombinant or genomic DNA expression. As used herein, the term "epitope" refers to the portion of an antigen (e.g., CLDN18.2) that specifically interacts with an antibody molecule.

A "complementarity determining region" or "CDR region" or "CDR" is a region in an antibody variable domain that is highly variable in sequence and forms structurally defined loops ("hypervariable loops") and/or contains antigen-contacting residues ("antigen-contacting sites"). The CDRs are primarily responsible for binding to antigenic epitopes. The CDRs of the heavy and light chains are generally referred to as CDR1, CDR2, and CDR3, and are numbered sequentially from the N-terminus. The CDRs located in the heavy chain variable domain of an antibody are referred to as HCDR1, HCDR2 and HCDR3, and the CDRs located in the light chain variable domain of an antibody are referred to as LCDR1, LCDR2 and LCDR3. In a given amino acid sequence of a light chain variable region or a heavy chain variable region, the exact amino acid sequence boundary of each CDR can be determined using any one or a combination of many well-known antibody CDR assignment systems including, for example, Chothia based on the three-dimensional structure of antibodies and the topology of CDR loops (Chothia et al. (1989) Nature 342:877-883, Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on affinity propagation clustering using a large number of crystal structures (North et al., "A New Clustering of Antibody CDR Loop Conformations", Journal of Molecular Biology, 406, 228-256 (2011)).

Table 1 below shows the region ranges of CDRs defined using the Kabat, AbM, Chothia, Contact, and IMGT schemes.

**Table 1:**

| CDR | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L52 |
| LCDR3 (Kabat and Chothia numbering systems) | L89-L97 | L89-L97 | L91-L96 | L89-L96 | L89-L96 |
| HCDR1 (Kabat numbering system) | H31-H35B | H26-H35B | H26-H32 | H30-H35B | H26-H35B |
| HCDR1 (Chothia numbering system) | H31-H35 | H26-H35 | H26-H32 | H30-H35 | H26-H35 |
| HCDR2 (Kabat and Chothia numbering systems) | H50-H65 | H50-H58 | H53-H55 | H47-H58 | H51-H57 |
| HCDR3 (Kabat and Chothia numbering systems) | H95-H102 | H95-H102 | H96-H101 | H93-H101 | H93-H102 |

CDRs can also be determined based on having the same Kabat numbering positions as a reference CDR sequence (e.g., any one of the exemplary CDRs of the present invention).

Unless otherwise stated, in the present invention, the term "CDR" or "CDR sequence" encompasses CDR sequences determined in any of the ways described above.

Unless otherwise stated, in the present invention, residue positions of an antibody variable region (including heavy chain variable region residues and light chain variable region residues) are numbered according to the Kabat numbering system (Kabat et al., Sequences of Proteins of Immunological Interest, 5th Ed. Public Health Service, National Institutes of Health, Bethesda, Md. (1991)).

In one embodiment, the CDRs of the heavy chain variable region of the antibody of the present invention are determined according to the following rules:
VH CDR1 is determined according to AbM rules; and VH CDR2 and VH CDR3 are both determined according to Kabat rules.

In one embodiment, the CDRs of the light chain variable region of the antibody of the present invention are determined according to the Kabat rules.

In one embodiment, the heavy chain variable region CDRs of the antibody of the present invention are determined according to the following rules: VH CDR1 is determined according to AbM rules; VH CDR2 and VH CDR3 are both determined according to Kabat rules; and the light chain variable region CDRs are determined according to Kabat rules.

It should be noted that the boundaries of the CDRs of the variable regions of the same antibody obtained based on different assignment systems may differ. That is, the CDR sequences of the variable regions of the same antibody defined under different assignment systems are different. Thus, when it comes to defining an antibody with specific CDR sequences defined in the present invention, the scope of the antibody also encompasses antibodies whose variable region sequences comprise the specific CDR sequences, but whose claimed CDR boundaries differ from the specific CDR boundaries defined in the present invention due to the application of different schemes (e.g., different assignment system rules or combinations).

Antibodies with different specificities (i.e., different binding sites for (the same or different) antigens) have different CDRs (under the same assignment system). However, although CDRs vary from antibody to antibody, only a limited number of amino acid positions within CDRs are directly involved in antigen binding. The smallest overlapping region can be determined using at least two of the Kabat, Chothia, AbM, Contact, and North methods, thereby providing a "minimal binding unit" for antigen binding. The minimal binding unit may be one sub-portion of the CDR. As will be appreciated by those skilled in the art, the residues in the rest of the CDR sequences can be determined by the structure and protein folding of the antibody. Thus, variants of any of the CDRs set forth herein are also contemplated by the present invention. For example, in a variant of one CDR, the amino acid residue of the smallest binding unit may remain unchanged, while the remaining CDR residues defined according to Kabat or Chothia may be substituted with conservative amino acid residues.

The term "Fc region" is used herein to define the C-terminal region of an immunoglobulin heavy chain that contains at least a portion of the constant region. The term includes native sequence Fc regions and variant Fc regions. A native immunoglobulin "Fc domain" comprises two or three constant domains, i.e., a CH2 domain, a CH3 domain, and optionally a CH4 domain. For example, in native antibodies, the immunoglobulin Fc domain comprises the second and third constant domains (CH2 domain and CH3 domain) derived from two heavy chains of IgG, IgA, and IgD antibodies; or comprises the second, third, and fourth constant domains (CH2 domain, CH3 domain, and CH4 domain) derived from two heavy chains of IgM and IgE antibodies. Unless otherwise stated herein, amino acid residues in the Fc region or heavy chain constant region are numbered according to the EU numbering system (also known as the EU index) as described in Kabat et al., Sequences of Proteins of Immunological Interes, 5th Ed., Public Health Service, National Institutes of Health, Bethesda, MD, 1991. Herein, the term "Fc region" does not include the heavy chain variable region VH and the light chain variable region VL as well as the heavy chain constant region CH1 and the light chain constant region CL of an immunoglobulin, but in some cases, may include a hinge region at the N-terminus of the heavy chain constant region.

"Antibody in IgG form" refers to the IgG form to which the heavy chain constant region of the antibody belongs. The heavy chain constant regions of all antibodies of the same type are identical, and the heavy chain constant regions of antibodies of different types are different. For example, an antibody in the form of IgG4 refers to that its heavy chain constant region is from IgG4, or an antibody in the form of IgG1 refers to that its heavy chain constant region is from IgG1.

As used herein, the term "binds" or "specifically binds" means that the binding effect is selective for an antigen and can be distinguished from unwanted or non-specific interactions. The ability of an antigen-binding site to bind to a particular antigen can be determined by enzyme-linked immunosorbent assay (ELISA) or conventional binding assays known in the art, such as radioimmunoassay (RIA), bio-layer interferometry, MSD assay, or surface plasmon resonance (SPR).

As used herein, "antibody-drug conjugate (ADC)" refers to a structure obtained by linking an antibody to a drug.

The term "drug-to-antibody ratio" or "DAR" refers to the ratio of a small molecule drug moiety (D) conjugated to an antibody (Ab) moiety described herein to the Ab moiety. In some embodiments described herein, the DAR may be determined by p and r in formula I; for example, the DAR may be 1 to 20, e.g., 2-18, 4-16, 5-12, 6-10, 2-8, 3-8, 2-6, 4-6, or 6-10, e.g., 2, 3, 4, 5, 6, 7, 8, 9, 10, 11, 12, 13, 14, or 15. The DAR may also be calculated as the average DAR of the molecular population in the product, i.e., the overall ratio of the small molecule drug moiety (D) (e.g., exatecan) conjugated to the Ab moiety described herein to the Ab moiety in the product as measured by detection methods (e.g., by conventional methods such as mass spectrometry, ELISA assay, electrophoresis, and/or HPLC), and this DAR is referred to herein as the average DAR. In some embodiments, the average DAR value of the conjugate of the present invention is 1-20, e.g., 2-18, 4-16, 5-12, 6-10, 2-8, 3-8, 2-6, 4-6, or 6-10, e.g., 1.0-8.0 or 2.0-6.0, e.g., 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10, or a range defined by any two of these values as endpoints, e.g., q is 2-5, e.g., q is 3-5 or 3.5-4.5. More specifically, q is 2, 2.5, 3, 3.5, 4, 4.8, or 5; or q is 5-11, such as 7-9 or 7.5-8.5.

The term "effective amount" refers to an amount or dose of the antibody or antibody fragment (e.g., antigen-binding fragment) or composition or combination of the present invention that produces the desired effect in a patient in need of treatment or prevention after administration to the patient in a single dose or multiple doses.

A "therapeutically effective amount" refers to an amount effective, at dosages and for periods of time necessary, to achieve the desired therapeutic result. A therapeutically effective amount is also one in which any toxic or detrimental effects of the antibody or antibody fragment (e.g., antigen binding fragment) or composition or combination are inferior to the therapeutically beneficial effects. A "therapeutically effective amount" preferably inhibits a measurable parameter (e.g., tumor volume) by at least about 30%, and even more preferably by at least about 40%, 45%, 50%, 55%, 60%, 65%, 70%, 75%, 80%, 85%, 90%, or even 100%, relative to untreated subjects.

The term "prophylactically effective amount" refers to an amount effective, at dosages and for periods of time necessary, to achieve the desired prophylactic result. Generally, since a prophylactic dose is used in a subject prior to or at an earlier stage of disease, the prophylactically effective amount will be less than the therapeutically effective amount.

The term "single drug dose unit" refers to a single drug dosage form, such as an injection, comprising a therapeutically effective ingredient (such as the Claudin18.2-targeted antibody-drug conjugate and/or the antibody drug and/or the chemotherapy drug according to the present invention) to be administered to a subject (preferably per kg body weight of the subject) at the time of a regimen, for example, placed in an ampoule (or vial).

The term "unit formulation" refers to the smallest administration unit or smallest packaging unit that can be separated in a pharmaceutical product. For example, where it is desired to administer 200 mg of a drug to an individual, a unit formulation of the drug may be a 10 mg packaging unit, whereby 20 packaging units of the drug are administered to the individual, or a 100 mg packaging unit, whereby 2 packaging units of the drug are administered to the individual, as desired. The packaging units exemplified herein, such as 10 mg or 100 mg, are for illustrative purposes only and are not intended to be limiting.

The terms "host cell", "host cell line", and "host cell culture" are used interchangeably and refer to cells into which exogenous nucleic acid has been introduced, including the progeny of such cells. Host cells include "transformants" and "transformed cells", which include primary transformed cells and progeny derived therefrom, regardless of the number of passages. Progeny may not be completely identical in nucleic acid content to the parent cell, and may contain mutations. The term as used herein includes mutant progeny having the same function or biological activity that are screened or selected from the initially transformed cells.

As used herein, the term "label" refers to a compound or composition that is directly or indirectly conjugated or fused to an agent, such as a polynucleotide probe or an antibody, and facilitates detection of the agent to which it is conjugated or fused. The label itself may be detectable (e.g., radioisotope labels or fluorescent labels) or, in the case of an enzymatic label, may catalyze chemical alteration of a substrate compound or composition that is detectable. The term is intended to encompass direct labeling of a probe or antibody by coupling (i.e., physically linking) a detectable substance to the probe or antibody, as well as indirect labeling of a probe or antibody by reaction with another reagent that is directly labeled.

"Individual" or "subject" or "object" includes mammals. Mammals include, but are not limited to, domestic animals (e.g., cattle, sheep, cats, dogs, and horses), primates (e.g., humans and non-human primates such as monkeys), rabbits, and rodents (e.g., mice and rats). In some embodiments, the individual or subject or object is a human.

An "isolated" antibody or other molecule (e.g., an ADC molecule) is one that has been separated from components of its natural environment or the environment in which it is expressed and synthesized. In some embodiments, the antibody or ADC molecule is purified to a purity greater than 95% or 99%, as determined by, for example, electrophoresis (e.g., SDS-PAGE, isoelectric focusing (IEF), capillary electrophoresis) or chromatography (e.g., ion exchange or reverse-phase HPLC).

The term "anti-tumor effect" refers to a biological effect that can be exhibited by a variety of means, including but not limited to, for example, a decrease in tumor volume, a decrease in the number of tumor cells, a decrease in tumor cell proliferation, or a decrease in tumor cell survival.

The terms "tumor" and "cancer" are used interchangeably herein to encompass solid tumors and hematological tumors.

The terms "cancer" and "cancerous" refer to or describe a physiological condition in mammals that is typically characterized by unregulated cell growth. In certain embodiments, cancers suitable for treatment by the antibody of the present invention include gastric cancer, pancreatic cancer, or gastroesophageal junction cancer, including metastatic forms of those cancers.

The term "tumor" refers to all neoplastic cell growth and proliferation, whether malignant or benign, and all pre-cancerous and cancerous cells and tissues. The terms "cancer", "cancerous" and "tumor" are not mutually exclusive when referred to herein.

As used herein, "treat", "treating" or "treatment" refers to slowing, interrupting, arresting, alleviating, stopping, reducing, or reversing the progression or severity of an existing symptom, disorder, condition, or disease.

As used herein, "prevention" includes the inhibition of the development or progression of a disease or disorder or a symptom of a particular disease or disorder. In some embodiments, a subject with a family history of cancer is a candidate for a prophylactic regimen. Generally, in the context of cancer, the term "prevention" refers to the administration of a drug prior to the onset of signs or symptoms of cancer, particularly in a subject at risk for cancer.

The term "vector," as used herein, refers to a nucleic acid molecule capable of propagating another nucleic acid to which it is linked. The term includes vectors that are self-replicating nucleic acid structures as well as vectors that are incorporated into the genome of a host cell into which they have been introduced. Some vectors are capable of directing the expression of a nucleic acid to which they are operably linked. Such vectors are referred to herein as "expression vectors".

A "subject/patient/individual sample" refers to a collection of cells or fluids obtained from a patient or subject. The source of the tissue or cell sample may be solid tissue, like from a fresh, frozen and/or preserved organ or tissue sample or biopsy or puncture sample; blood or any blood component; body fluids, such as cerebrospinal fluid, amniotic fluid, peritoneal fluid (ascites), or interstitial fluid; cells from any time in gestation or development of the subject. The tissue sample may contain compounds that are not naturally intermixed with the tissue in nature, such as preservatives, anticoagulants, buffers, fixatives, nutrients, antibiotics, and the like.

### Methods of treatment

The present invention provides a method of using a Claudin18.2-targeted antibody-drug conjugate in a combination therapy for cancer, comprising administering to an individual in need thereof an effective amount of the Claudin18.2-targeted antibody-drug conjugate and an antibody drug for treating cancer and/or a chemotherapy drug.

In some embodiments of the present invention, the Claudin18.2-targeted antibody-drug conjugate used is selected from: 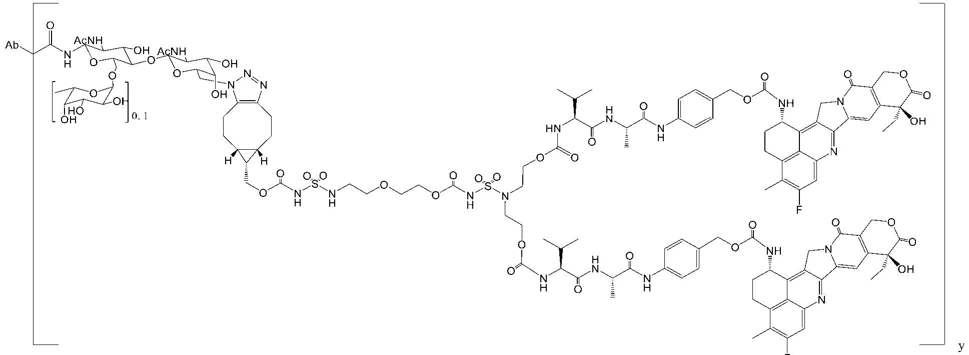
wherein Ab is a Claudin18.2-targeted antibody or an antigen-binding fragment thereof;
wherein y is an integer of 1 or 2.

In some embodiments of the present invention, the Claudin18.2-targeted antibody-drug conjugate used is selected from: 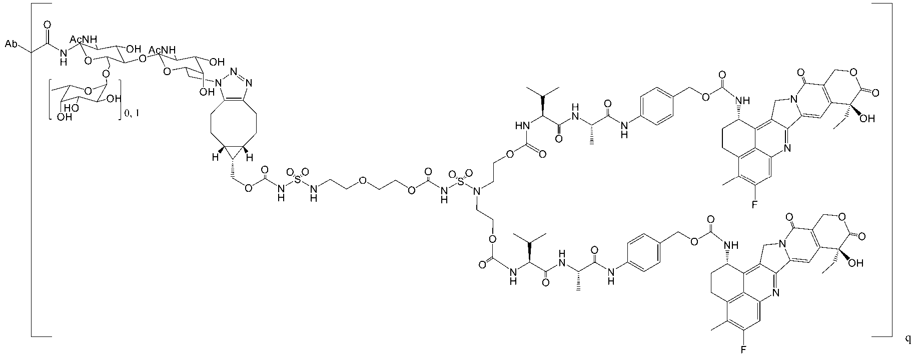 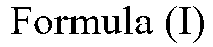
wherein Ab is a Claudin18.2-targeted antibody or an antigen-binding fragment thereof;
q represents an average DAR, and q is 2-11; wherein q is 2-5, for example, q is 3-5 or 3.5-4.5, and more specifically, q is 2, 2.5, 3, 3.5, 4, 4.8 or 5; or q is 5-11, for example, 7-9 or 7.5-8.5.

In some embodiments of the present invention, the Claudin18.2-targeted antibody or the antigen-binding fragment thereof comprises HCDR1, HCDR2 and HCDR3 set forth in amino acid sequences of SEQ ID NO: 1 (GFTFSSYVMS), SEQ ID NO: 2 (TISHSGGSTYYADSVKG) and SEQ ID NO: 3 (DAPYYDILTGYRY), respectively, and LCDR1, LCDR2 and LCDR3 set forth in amino acid sequences of SEQ ID NO: 6 (RASQSISSWLA), SEQ ID NO: 7 (KASSLES) and SEQ ID NO: 8 (QQYNSYSYT).

In some embodiments of the present invention, the Claudin18.2-targeted antibody or the antigen-binding fragment thereof comprises a heavy chain variable region and/or a light chain variable region, wherein the heavy chain variable region
(i) comprises or consists of an amino acid sequence set forth in SEQ ID NO: 4; or
(ii) comprises or consists of an amino acid sequence having one or more (preferably no more than 10, and more preferably no more than 5, 4, 3, 2, or 1) amino acid changes (preferably amino acid substitutions, and more preferably conservative amino acid substitutions) compared to an amino acid sequence set forth in SEQ ID NO: 4, wherein preferably, the amino acid changes do not occur in the CDR regions; or
(iii) comprises or consists of an amino acid sequence having at least 85%, at least 90%, at least 91%, at least 92%, at least 93%, at least 94%, at least 95%, at least 96%, at least 97%, at least 98%, or at least 99% identity to the amino acid sequence set forth in SEQ ID NO: 4; and/or
the light chain variable region
(i) comprises or consists of an amino acid sequence set forth in SEQ ID NO: 9; or
(ii) comprises or consists of an amino acid sequence having one or more (preferably no more than 10, and more preferably no more than 5, 4, 3, 2, or 1) amino acid changes (preferably amino acid substitutions, and more preferably conservative amino acid substitutions) compared to an amino acid sequence set forth in SEQ ID NO: 9, wherein preferably, the amino acid changes do not occur in the CDR regions; or
(iii) comprises or consists of an amino acid sequence having at least 85%, at least 90%, at least 91%, at least 92%, at least 93%, at least 94%, at least 95%, at least 96%, at least 97%, at least 98%, or at least 99% identity to the amino acid sequence set forth in SEQ ID NO: 9.

In some embodiments of the present invention, the Claudin18.2-targeted antibody or the antigen-binding fragment thereof of the present invention comprises a heavy chain constant region. In some embodiments, the Claudin18.2-targeted antibody or the antigen-binding fragment thereof of the present invention comprises a light chain constant region. In some embodiments, the Claudin18.2-targeted antibody or the antigen-binding fragment thereof of the present invention further comprises a heavy chain constant region and a light chain constant region.

In some embodiments of the present invention, the heavy chain constant region (HC) of the present invention is a heavy chain constant region of IgG1, IgG2, IgG3, or IgG4, preferably a heavy chain constant region of IgG1, such as a heavy chain constant region of wild-type IgG1. In some embodiments, the antibody light chain constant region (LC) of the present invention is a lambda or Kappa light chain constant region.

In some preferred embodiments, the heavy chain constant region (HC) of the present invention
(i) comprises or consists of an amino acid sequence having at least 85%, at least 90%, at least 91%, at least 92%, at least 93%, at least 94%, at least 95%, at least 96%, at least 97%, at least 98%, or at least 99% identity to an amino acid sequence set forth in SEQ ID NO: 5;
(ii) comprises or consists of an amino acid sequence set forth in SEQ ID NO: 5; or
(iii) comprises or consists of an amino acid sequence having one or more (preferably no more than 20 or 10, and more preferably no more than 5, 4, 3, 2, or 1) amino acid changes (preferably amino acid substitutions, and more preferably conservative amino acid substitutions) compared to the amino acid sequence set forth in SEQ ID NO: 5.

In some preferred embodiments, the light chain constant region (LC) of the antibody of the present invention
(i) comprises or consists of an amino acid sequence having at least 85%, at least 90%, at least 91%, at least 92%, at least 93%, at least 94%, at least 95%, at least 96%, at least 97%, at least 98%, or at least 99% identity to an amino acid sequence set forth in SEQ ID NO: 10;
(ii) comprises or consists of an amino acid sequence set forth in SEQ ID NO: 10; or
(iii) comprises or consists of an amino acid sequence having one or more (preferably no more than 20, and more preferably no more than 5, 4, 3, 2, or 1) amino acid changes (preferably amino acid substitutions, and more preferably conservative amino acid substitutions) compared to the amino acid sequence set forth in SEQ ID NO: 10.

In some embodiments of the present invention, the Claudin18.2-targeted antibody or the antigen-binding fragment thereof of the present invention comprises a heavy chain and a light chain, wherein the heavy chain comprises or consists of an amino acid sequence of SEQ ID NO: 11 shown below: 
EVQLLDSGGGLVQPGGSLRLSCAASGFTFSSYVMSWVRQAPGKGLNWVSTISHSGGSTYYADSVKGR
FTISRDNSKNTLYLQMNSLRAEDTAVYYCAIDAPYYDILTGYRYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTISKAKGQPREPQ
VYTLPPSRDELTKNQVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO:11);
 or
comprises or consists of an amino acid sequence having one or more (preferably no more than 20 or 10, and more preferably no more than 5, 4, 3, 2, or 1) amino acid changes (preferably amino acid substitutions, and more preferably conservative amino acid substitutions) compared to an amino acid sequence set forth in SEQ ID NO: 11,
   or
comprises or consists of an amino acid sequence having at least 85%, at least 90%, at least 91%, at least 92%, at least 93%, at least 94%, at least 95%, at least 96%, at least 97%, at least 98%, or at least 99% identity to an amino acid sequence of SEQ ID NO: 11; and
the light chain comprises or consists of an amino acid sequence set forth in SEQ ID NO: 12 shown below: 
DIQMTQSPSTLSASVGDRVTITCRASQSISSWLAWYQQKPGKAPKLLIYKASSLESGVPSRFSGSGSGTE
FTLTISSLQPDDFATYYCQQYNSYSYTFGQGTKLEIKRTVAAPSVFIFPPSDEQLKSGTASVVCLLNNFYPRE
AKVQWKVDNALQSGNSQESVTEQDSKDSTYSLSSTLTLSKADYEKHKVYACEVTHQGLSSPVTKSFNRGE
C (SEQ ID NO: 12);
 or
comprises or consists of an amino acid sequence having one or more (preferably no more than 20 or 10, and more preferably no more than 5, 4, 3, 2, or 1) amino acid changes (preferably amino acid substitutions, and more preferably conservative amino acid substitutions) compared to an amino acid sequence set forth in SEQ ID NO: 12,
   or
comprises or consists of an amino acid sequence having at least 85%, at least 90%, at least 91%, at least 92%, at least 93%, at least 94%, at least 95%, at least 96%, at least 97%, at least 98%, or at least 99% identity to an amino acid sequence of SEQ ID NO: 12.

In some embodiments of the present invention, the method of the present invention comprises administering to an individual the Claudin18.2-targeted antibody-drug conjugate at a dose of 0.1 mg/kg-100 mg/kg; further, the method comprises administering to an individual the Claudin18.2-targeted antibody-drug conjugate at a dose of 0.2 mg/kg-50 mg/kg; still further, the method comprises administering to an individual the Claudin18.2-targeted antibody-drug conjugate at a dose of 0.3 mg/kg-30 mg/kg. For example, the method comprises administering to an individual the Claudin18.2-targeted antibody-drug conjugate at 0.3 mg/kg, 1 mg/kg, 3 mg/kg, 4.5 mg/kg, 6 mg/kg, 7.5 mg/kg, 8 mg/kg, 10 mg/kg, 11 mg/kg, 12 mg/kg, 13.5 mg/kg, 15 mg/kg. Still further, the method comprises administering to the individual the Claudin18.2-targeted antibody-drug conjugate at 1 mg/kg-30 mg/kg. For example, the method comprises administering to an individual the Claudin18.2-targeted antibody-drug conjugate at 1 mg/kg, 3 mg/kg, 4.5 mg/kg, 6 mg/kg, 7.5 mg/kg, 8 mg/kg, 10 mg/kg, 12 mg/kg, 13.5 mg/kg, 15 mg/kg, 16 mg/kg, 18 mg/kg, 20 mg/kg, 22 mg/kg, 25 mg/kg, 28 mg/kg, or 30 mg/kg.

In some embodiments of the present invention, the administration cycle of the method is 2 weeks/14 days (Q2W) - 5 weeks/35 days (Q5W); further, the administration cycle of the method is 2 weeks/14 days (Q2W), 3 weeks/21 days (Q3W), 4 weeks/28 days (Q4W), or 5 weeks/35 days (Q5W), and the administration is performed once on day 1 of each cycle.

In some embodiments of the present invention, the antibody drug for treating cancer is selected from one or a combination of more of a PD-1 inhibitor, a recombinant anti-VEGF monoclonal antibody, and a monoclonal antibody that specifically binds to VEGFR-2.

In some embodiments, the PD-1 inhibitor is selected from one or a combination of more of pembrolizumab, nivolumab, camrelizumab, sintilimab, toripalimab, and tislelizumab.

In one specific embodiment of the present invention, the method comprises administering to an individual in need thereof 200 mg of sintilimab. In a specific embodiment of the present invention, the administration cycle of sintilimab is 3 weeks/21 days (Q3W), e.g., administration on day 1.

In some embodiments of the present invention, the recombinant anti-VEGF monoclonal antibody is selected from one or a combination of two of sevacizumab and bevacizumab.

In a specific embodiment of the present disclosure, the method comprises administering to an individual in need thereof 15 mg/kg of bevacizumab. In a specific embodiment of the present invention, the administration cycle of bevacizumab is 3 weeks/21 days (Q3W), e.g., administration on day 1.

In some embodiments of the present invention, the monoclonal antibody that specifically binds to VEGFR-2 comprises ramucirumab.

In some specific embodiments of the present invention, the method comprises administering to an individual in need thereof 8 mg/kg of ramucirumab. In some specific embodiments of the present disclosure, the administration cycle of ramucirumab is 4 weeks/28 days (Q4W), e.g., administration on day 1 and day 15.

In some embodiments of the present invention, the chemotherapy drug is selected from one or a combination of more of oxaliplatin, capecitabine, gemcitabine, albumin-bound paclitaxel, and tegafur, gimeracil and oteracil potassium.

In some specific embodiments, the administration dose of oxaliplatin is 130 mg/m²; the administration cycle of oxaliplatin is 3 weeks/21 days (Q3W), e.g., administration on day 1.

In some specific embodiments, the administration dose of capecitabine is 1000 mg/m²; the administration cycle of capecitabine is twice daily.

In some specific embodiments, the administration dose of gemcitabine is 1000 mg/m²; the administration cycle of gemcitabine is 3 weeks/21 days (Q3W), e.g., administration on day 1 and day 8.

In some specific embodiments, the administration dose of the albumin-bound paclitaxel is 125 mg/m²; the administration cycle of the albumin-bound paclitaxel is 3 weeks/21 days (Q3W), e.g., administration on day 1 and day 8.

In some specific embodiments, the administration dose of tegafur, gimeracil and oteracil potassium is 40 mg-60 mg; the administration cycle of tegafur, gimeracil and oteracil potassium is 3 weeks/21 days (Q3W), twice daily, e.g., administration on days 1-14.

In some specific embodiments, the method of using the Claudin18.2-targeted antibody-drug conjugate of the invention in a combination therapy for cancer comprises:
Mode 1: the Claudin18.2-targeted antibody-drug conjugate of the present invention is administered at 4 mg/kg - 10 mg/kg (preferably 6 mg/kg), every 21 days as a cycle, administration on day 1; ramucirumab is administered at 8 mg/kg, every 28 days as a cycle, administration on day 1 and day 15.
Mode 2: the Claudin18.2-targeted antibody-drug conjugate of the present invention is administered at 4 mg/kg - 10 mg/kg (preferably 6 mg/kg), every 21 days as a cycle, administration on day 1; bevacizumab is administered at 15 mg/kg, every 21 days as a cycle, administration on day 1.
Mode 3: the Claudin18.2-targeted antibody-drug conjugate of the present invention is administered at 4 mg/kg - 10 mg/kg (preferably 6 mg/kg), every 21 days as a cycle, administration on day 1 of each cycle; sintilimab is administered at 200 mg, every 21 days as a cycle, administration on day 1.
Mode 4: the Claudin18.2-targeted antibody-drug conjugate of the present invention is administered at 4 mg/kg - 10 mg/kg (preferably 6 mg/kg), every 21 days as a cycle, administration on day 1 of each cycle; sintilimab is administered at 200 mg, every 21 days as a cycle, administration on day 1; oxaliplatin is administered at 130 mg/m², every 21 days as a cycle, administration on day 1. Each subject will receive up to 6 cycles of oxaliplatin treatment.
Mode 5: the Claudin18.2-targeted antibody-drug conjugate of the present invention is administered at 4 mg/kg - 10 mg/kg (preferably 6 mg/kg), every 21 days as a cycle, administration on day 1 of each cycle; sintilimab is administered at 200 mg, every 21 days as a cycle, administration on day 1; capecitabine is administered at 1000 mg/m² twice daily, every 21 days as a cycle, administration on days 1-14; oxaliplatin is administered at 130 mg/m² , every 21 days as a cycle, administration on day 1 (oxaliplatin will be administered for up to 6 cycles).
Mode 6: the Claudin18.2-targeted antibody-drug conjugate of the present invention is administered at 4 mg/kg - 10 mg/kg (preferably 6 mg/kg), every 21 days as a cycle, administration on day 1 of each cycle; tegafur, gimeracil and oteracil potassium is administered at 40 mg - 60 mg twice daily, every 21 days as a cycle, administration on days 1-14.
Mode 7: the Claudin18.2-targeted antibody-drug conjugate of the present invention is administered at 4 mg/kg - 10 mg/kg (preferably 6 mg/kg), every 21 days as a cycle, administration on day 1 of each cycle; gemcitabine is administered at 1000 mg/m², every 21 days as a cycle, administration on day 1 and day 8; and albumin-bound paclitaxel is administered at 125 mg/m², every 21 days as a cycle, administration on day 1 and day 8.

In some preferred embodiments of the present invention, the method of using the Claudin18.2-targeted antibody-drug conjugate of the invention in a combination therapy for cancer may comprise the modes shown in Table 2:

**Table 2**

| **Drug Product** | **Dose/Amount** | **Frequency of Administration** | **Usage Method** | **Treatment Course/Treatment Cycle** | |
|---|---|---|---|---|---|
| Claudin18.2-Targeted Antibody-Drug Conjugate of the Present Invention | 6 mg/kg; | Q3W | Intravenous infusion | Every 21 days as a cycle | **Mode 1** |
| | | | | Administration on Day 1 | |
| Ramucirumab | 8 mg/kg | Q4W | Intravenous infusion | Every 28 days as a cycle | |
| | | | | Administration on Day 1 and Day 15 | |
| | | | | | |
| Claudin18.2-Targeted Antibody-Drug Conjugate of the Present Invention | 6 mg/kg; | Q3W | Intravenous infusion | Every 21 days as a cycle | **Mode 2** |
| | | | | Administration on Day 1 | |
| Bevacizumab | 15 mg/kg | Q3W | Intravenous infusion | Every 21 days as a cycle | |
| | | | | Administration on Day 1 | |
| | | | | | |
| Claudin18.2-Targeted Antibody-Drug Conjugate of the Present Invention | 6 mg/kg; | Q3W | Intravenous infusion | Every 21 days as a cycle | **Mode 3** |
| | | | | Administration on Day 1 | |
| Sintilimab | 200 mg | Q3W | Intravenous infusion | Every 21 days as a cycle | |
| | | | | Administration on Day 1 | |
| | | | | | |
| Claudin18.2-Targeted Antibody-Drug Conjugate of the Present Invention | 6 mg/kg; | Q3W | Intravenous infusion | Every 21 days as a cycle | **Mode 4** |
| | | | | Administration on Day 1 | |
| Sintilimab | 200 mg | Q3W | Intravenous infusion | Every 21 days as a cycle | |
| | | | | Administration on Day 1 | |
| Oxaliplatin * | 130 mg/m² | Q3W | Intravenous infusion | Every 21 days as a cycle | |
| | | | | Administration on Day 1 | |
| *Each subject will receive up to 6 cycles of oxaliplatin treatment. | | | | | |
| | | | | | |
| Claudin18.2-Targeted Antibody-Drug Conjugate of the Present Invention | 6 mg/kg; | Q3W | Intravenous infusion | Every 21 days as a cycle | **Mode 5** |
| | | | | Administration on Day 1 | |
| Sintilimab | 200 mg | Q3W | Intravenous infusion | Every 21 days as a cycle | |
| | | | | Administration on Day 1 | |
| Oxaliplatin * | 130 mg/m² | Q3W | Intravenous infusion | Every 21 days as a cycle | |
| | | | | Administration on Day 1 | |
| Capecitabine | 1000 mg/m² | BID | Oral administration | Every 21 days as a cycle | |
| | | | | Administration on Days 1-14 | |
| *Each subject will receive up to 6 cycles of oxaliplatin treatment. | | | | | |
| | | | | | |
| Claudin18.2-Targeted Antibody-Drug Conjugate of the Present Invention | 6 mg/kg; | Q3W | Intravenous infusion | Every 21 days as a cycle | **Mode 6** |
| | | | | Administration on Day 1 | |
| Tegafur, gimeracil and oteracil potassium | 40 mg-60 mg | BID | Oral administration | Every 21 days as a cycle | |
| | | | | Administration on Days 1-14 | |
| | | | | | |
| Claudin18.2-Targeted Antibody-Drug Conjugate of the Present Invention | 6 mg/kg; | Q3W | Intravenous infusion | Every 21 days as a cycle | **Mode 7** |
| | | | | Administration on Day 1 | |
| Gemcitabine | 1000 mg/m² | Q3W | Intravenous infusion | Every 21 days as a cycle | |
| | | | | Administration on Day 1 and Day 8 | |
| Albumin-bound paclitaxel | 125 mg/m² | Q3W | Intravenous infusion | Every 21 days as a cycle | |
| | | | | Administration on Day 1 and Day 8 | |

In some embodiments of the present invention, in the method of using the Claudin18.2-targeted antibody-drug conjugate of the invention in a combination therapy for cancer, the cancer is a solid tumor; further, the solid tumor may be a Claudin18.2-related tumor, such as gastric cancer, breast cancer, liver cancer, colon cancer, lung cancer, gallbladder cancer, esophageal cancer, gastric/gastroesophageal junction adenocarcinoma, pancreatic ductal adenocarcinoma, and biliary tract cancer (biliary tract cancer is divided into cholangiocarcinoma and gallbladder cancer). In some preferred embodiments, the Claudin18.2-targeted antibody-drug conjugate of the present invention is used in a combination therapy for treating gastric/gastroesophageal junction adenocarcinoma. In a preferred embodiment of the present invention, the Claudin18.2-targeted antibody-drug conjugate of the present invention is used in a combination therapy for treating pancreatic ductal adenocarcinoma.

In some specific embodiments, the Claudin18.2-targeted antibody-drug conjugate of the present invention is administered in combination with sintilimab, ramucirumab, or bevacizumab, respectively, for treating gastric/gastroesophageal junction adenocarcinoma. In some preferred embodiments, the Claudin18.2-targeted antibody-drug conjugate of the present invention is administered in combination with sintilimab, ramucirumab, or bevacizumab, respectively, in the treatment of CLDN18.2-positive advanced gastric/gastroesophageal junction adenocarcinoma that has failed or is intolerant to first-line standard treatment.

In some specific embodiments, the Claudin18.2-targeted antibody-drug conjugate of the present invention is used in combination with sintilimab and/or capecitabine and/or oxaliplatin to treat gastric/gastroesophageal junction adenocarcinoma. In some preferred embodiments, the Claudin18.2-targeted antibody-drug conjugate of the present invention is used in combination with sintilimab and/or capecitabine and/or oxaliplatin to treat HER2-negative and CLDN18.2-positive gastric/gastroesophageal junction adenocarcinoma that has not been treated systemically.

In some specific embodiments, the Claudin18.2-targeted antibody-drug conjugate of the present invention is used in combination with tegafur, gimeracil and oteracil potassium to treat pancreatic ductal adenocarcinoma. In some preferred embodiments, the Claudin18.2-targeted antibody-drug conjugate of the present invention is used in combination with tegafur, gimeracil and oteracil potassium to treat CLDN18.2-positive pancreatic ductal adenocarcinoma that has failed or is intolerant to the previous treatment with a gemcitabine-based regimen.

In some specific embodiments, the Claudin18.2-targeted antibody-drug conjugate of the present invention is used in combination with gemcitabine and albumin-bound paclitaxel to treat pancreatic ductal adenocarcinoma. In some preferred embodiments, the Claudin18.2-targeted antibody-drug conjugate of the present invention is used in combination with gemcitabine and albumin-bound paclitaxel to treat CLDN18.2-positive pancreatic ductal adenocarcinoma that has not been treated systematically.

In some specific embodiments of the present invention, the administration mode of the method of the present invention comprises intravenous injection and oral administration. In some specific embodiments, the Claudin18.2-targeted antibody-drug conjugate of the present invention, sintilimab, ramucirumab, bevacizumab, oxaliplatin, albumin-bound paclitaxel, and gemcitabine are administered by intravenous injection. In some specific embodiments, capecitabine, tegafur, gimeracil and oteracil potassium are administered orally.

In some specific embodiments of the present invention, the active ingredient of the Claudin18.2-targeted antibody-drug conjugate of the present invention is a recombinant anti-Claudin18.2 monoclonal antibody-exatecan conjugate in the dosage form of a lyophilized formulation for injection with a specification of 100 mg/vial. In some specific embodiments of the present invention, the finished product of the Claudin18.2-targeted antibody-drug conjugate of the present invention comprises 20 mg/mL stock solution of the Claudin18.2-targeted antibody-drug conjugate of the present invention, histidine, histidine hydrochloride, sucrose and polysorbate 80. The Claudin18.2-targeted antibody-drug conjugate of the present invention should be prepared by professional health personnel using aseptic techniques. In some specific embodiments of the present invention, the finished product should be diluted with 5% glucose injection. In some specific embodiments of the present invention, the final concentration of the Claudin18.2-targeted antibody-drug conjugate of the present invention should be maintained at 1 mg/mL-6 mg/mL. Administration of the Claudin18.2-targeted antibody-drug conjugate of the present invention should be performed through an intravenous infusion tube equipped with a 0.2 µm-5 µm in-line filter.

### BRIEF DESCRIPTION OF THE DRAWINGS

FIG. 1 shows the anti-tumor efficacy of a CLAUDIN18.2-targeted antibody-drug conjugate in combination with an anti-PD-1 antibody in an EMT6-hClaudin18.2 tumor-bearing mouse model.
FIG. 2 shows the effect of a CLAUDIN18.2-targeted antibody-drug conjugate in combination with an anti-PD-1 antibody on the change in body weight of mice.
FIG. 3 shows the anti-tumor efficacy of a CLAUDIN18.2-targeted antibody-drug conjugate in combination with capecitabine and oxaliplatin in an HCT15-Claudin 18.2 tumor-bearing mouse model.
FIG. 4 shows the effect of a CLAUDIN18.2-targeted antibody-drug conjugate in combination with capecitabine and oxaliplatin on the body weight of HCT15-Claudin 18.2 tumor-bearing mice.

### DETAILED DESCRIPTION OF EMBODIMENTS

The embodiments of the present invention will be described in detail below with reference to the examples. Those skilled in the art will appreciate that the following examples are merely illustrative of the present invention and should not be construed as limiting the scope of the present invention. The examples without specified techniques or conditions were performed according to the techniques or conditions described in the literature in the art or according to the product instructions. Reagents or instruments without specified manufacturers used are commercially available conventional products.

### EXAMPLES

### Example 1: Preparation of Claudin18.2-Targeted Antibody-Drug Conjugates

The anti-CLDN18.2 monoclonal antibody HB37A6 (with a heavy chain sequence set forth in SEQ ID NO: 11 and a light chain sequence set forth in SEQ ID NO: 12; see CN202010570517.X) and the control antibody zolbetuximab (Zmab for short, with a sequence derived from International Nonproprietary Names for Pharmaceutical Substances (INN) 117) were expressed as full-length monoclonal antibodies in HEK293 cells (Invitrogen, A14527).

On the basis of HB37A6, an ADC was further designed and synthesized. For the specific synthesis steps, reference was made to the preparation steps of IEX019-02 in PCT/CN2022/139637 (Example 2.1.1), the contents of which are incorporated herein by reference.

### Example 2

20 mg/mL IEX019-02 ADC, 20 mM histidine solution, 8% (w/v) sucrose, and 0.02% (w/v) polysorbate 80, pH 6.5 were lyophilized (the lyophilization process was performed according to a conventional process in the art). The lyophilization process parameters are shown in Table 3.

**Table 3**

| Steps | Temperature/°C | Rate/min | Holding time/min | Pressure/mbar |
|---|---|---|---|---|
| Loading temperature | 5 | 30 | 60 | 1000 |
| Frozen | -20 | 40 | 120 | 1000 |
| | -45 | 90 | 180 | 1000 |
| | -7 | 40 | 180 | 1000 |
| | -45 | 40 | 180 | 1000 |
| Primary drying | -15 | 30 | 2520 | 0.1 |
| Secondary drying | 35 | 250 | 720 | 0.1 |

### Example 3

### 1. Test antibody and other drugs

Study drug: Claudin18.2-targeted antibody-drug conjugate (formulation of Example 2).
Specification: 100 mg/vial
Drug source: Innovent Biologics (Suzhou) Co., Ltd.
Route of administration: intravenous infusion (IV)
Sintilimab
Specification: 100 mg (10 mL)/vial
Route of administration: 200 mg Q3W intravenous infusion
Drug source: Innovent Biologics (Suzhou) Co., Ltd.
Ramucirumab
Specification: 100 mg (10 mL)/vial, 500 mg (50 mL)/vial
Route of administration: 8 mg/kg, every 28 days as a cycle, administration on day 1 and day 15 of each cycle, intravenous infusion
Drug source: Innovent Biologics (Suzhou) Co., Ltd.
Bevacizumab
Specification: 100 mg (4 mL)/vial
Route of administration: 15 mg/kg, Q3W, intravenous infusion
Drug source: Innovent Biologics (Suzhou) Co., Ltd.
Chemotherapy agents
The specifications and routes of administration of oxaliplatin, capecitabine, gemcitabine, albumin-bound paclitaxel, and tegafur, gimeracil and oteracil potassium are detailed in the protocol.

### 2. Purpose of the study

To evaluate the efficacy and safety of the Claudin18.2-targeted antibody-drug conjugate in combination with an antibody drug or a chemotherapy drug in the treatment of cancer subjects.

To evaluate the pharmacokinetic (PK) characteristics of the Claudin18.2-targeted antibody-drug conjugate, an anti-Claudin18.2 (CLDN18.2) antibody and Exatecan.

To evaluate the immunogenicity of the Claudin18.2-targeted antibody-drug conjugate.

### 3. Dose-limiting toxicity

Toxicity evaluation was performed according to the National Cancer Institute Common Terminology Criteria for Adverse Events (NCI CTCAE) v5.0. DLT was defined as any one of the following adverse events (AEs) associated with the study drug: a Claudin18.2-targeted antibody-drug conjugate within 21 days after the first dose in the dose finding phase:
1. Hematologic Toxicity
   - Grade 4 neutrophil count decreased lasting > 7 days
   - Grade ≥ 3 febrile neutropenia [absolute neutrophil count (ANC) < 1 × 10 ⁹ /L and a single temperature > 38.3 °C or a sustained temperature ≥ 38.0 °C for more than 1 h]
   - Grade 4 anemia not explained by underlying disease
   - Grade 4 platelet count decreased
   - ≥Grade 3 platelet count decreased lasting >7 days
   - ≥Grade 3 platelet count decreased with bleeding
   - Grade 4 lymphocyte count decreased lasting ≥ 14 days
2. Hepatotoxicity
   - Grade 4 aspartate aminotransferase (AST) or alanine aminotransferase (ALT) elevation
   - AST or ALT > 5 × upper limit of normal value (ULN) with ≥ grade 2 total bilirubin (TBIL) elevation
   - AST or ALT >5×ULN for >7 days following optimal treatment in subjects without liver metastases
   - In subjects with liver metastases, if baseline level is ≤ 3 × ULN and AST or ALT is > 5 × ULN, the event persists for > 7 days after optimal treatment
   - In subjects with liver metastases, if baseline level is > 3 × ULN and AST or ALT is > 8 × ULN, the event persists for > 7 days after optimal treatment
3. Non-hematologic and non-hepatotoxicity
   1) Any ≥ Grade 3 non-hematologic, non-major hepatotoxicity (except laboratory abnormalities), with the following exceptions:
      - Grade 3 arthralgia
      - Grade 3 myalgia
      - Grade 3 rash
      - Grade 3 alopecia
      - Grade 3 fatigue/asthenia
      - Grade 3 hypertension or hypotension
      - Grade 3 nausea, vomiting, diarrhea, or anorexia that resolves to ≤Grade 2 within 3 days with best supportive care
   2) Any ≥ Grade 3 non-hematologic, non-major hepatotoxic laboratory abnormality that requires treatment or leads to hospitalization, with the following exceptions:
      - Grade 3 electrolyte abnormalities
      - Grade 3 gamma-glutamyl transferase (GGT) elevation
      - Asymptomatic laboratory abnormalities including grade 3/4 alkaline phosphatase increased, hyperuricemia, serum amylase increased, and lipase increased
4. Any Grade 5 AE
5. Other AEs determined to be DLTs by the investigator and the sponsor through discussion

Note:
- Grade 3 or 4 infusion related reactions (IRRs) are not considered DLTs. However, if a Grade 3 or 4 IRR occurs, the subject needs to discontinue study treatment and be replaced with a new subject. If ≥ 2 subjects experience Grade 3 or 4 IRRs in a dose group, enrollment must be suspended. The sponsor needs to review the safety data of the study to determine whether to continue enrollment.
- Throughout the trial, the above criteria will be used to assess whether a subject needs dose delay or discontinuation. However, only DLTs that occur during the DLT observation period after the first dose will be used to determine dose escalation and tolerability.
- If any of the above toxicities occurs during the DLT observation period, the investigator and the sponsor will determine whether the toxicity is a DLT after consultation. In addition, for other toxicities that affect the treatment with the investigational drug, the investigator and the sponsor will determine whether the toxicity is a DLT after consultation.

### 4. Criteria for enrolling/excluding subjects

Subjects must meet all of the following conditions to participate in the study:
1. Signed the informed consent form (ICF), willing and able to comply with the visits and related procedures specified in the protocol.
2. At least 1 measurable lesion according to Response Evaluation Criteria in Solid Tumors RECIST v1.1 (without prior radiotherapy). (The long diameter is ≥ 10 mm as accurately measured by computed tomography (CT) or magnetic resonance imaging (MRI) (intravenous contrast is preferred) at baseline (except for lymph nodes, which must have a short axis ≥ 15 mm), the diameter of the target lesion is ≥ 2 times the slice thickness, and the lesion is suitable for repeated accurate measurement. Lesions located in a previously irradiated area may be considered as measurable lesions if there is clear evidence of progression per RECIST V1.1 standard).
3. Age ≥ 18 years old, gender is not limited.
4. Eastern Cooperative Oncology Group Performance Status (ECOG PS) of 0 or 1.
5. Expected survival ≥ 12 weeks.
6. Adequate bone marrow and organ function:
   a. Blood routine: ANC ≥ 1.5 × 10 ⁹ /L; platelet count ≥ 100 × 10 ⁹ /L; hemoglobin content ≥ 9.0 g/dL, and the subject must not have received transfusion of blood products (including red blood cell suspension, apheresis platelets, cryoprecipitate, etc.), erythropoietin (EPO), Granulocyte-colony stimulating factor (G-CSF), or granulocyte-macrophage colony-stimulating factor (GM-CSF) within 7 days prior to blood sampling;
   b. Liver function: TBIL ≤ 1.5 × ULN (TBIL ≤ 3 × ULN is allowed for subjects with Gilbert syndrome); ALT and AST ≤ 2.5 × ULN for subjects without liver metastasis, and ALT and AST ≤ 5 × ULN for subjects with liver metastasis; albumin ≥ 28 g/L;
   c. Renal function: creatinine clearance ≥ 60 mL/min (Cockcroft-Gault formula); urine protein < 2+ or total 24-h urine protein < 1 g;
   d. Coagulation function: International Normalized Ratio (INR) ≤ 1.5 and activated partial thromboplastin time (APTT) ≤ 1.5 × ULN (subjects with anticoagulant therapy and coagulation function within the above range are allowed).
7. Female subjects of childbearing age or male subjects whose partners are women of childbearing age must take effective contraceptive measures during the entire treatment period and within 6 months after the treatment period.
8. Pathological tissue detection confirmed *CLDN18.2-positive.

### Cohort A:

9. Histopathologically confirmed unresectable locally advanced, recurrent or metastatic G/GEJ AC.
10. Previously received first-line systemic standard treatment and failed or was not tolerated.

### Cohort B:

11. Histopathologically confirmed unresectable locally advanced, recurrent or metastatic HER2-negative G/GEJ AC.
12. Subjects must not be suitable for radical treatment methods, such as radical chemoradiotherapy and/or surgery; for subjects who have previously received (neo) adjuvant chemotherapy/adjuvant (radiotherapy) chemotherapy/radical radiochemotherapy, the time from the last treatment to disease recurrence is > 6 months.
13. Patients who have not received any systemic treatment (including chemotherapy, targeted therapy, tumor immunotherapy, etc.) in the locally advanced or metastatic stage.

Cohort C:
14. Histopathologically confirmed unresectable locally advanced, recurrent or metastatic PDAC.
15. Previously received at least first-line gemcitabine-based systemic standard therapy and has failed or is intolerant to the therapy;

### Cohort D:

16. Histopathologically confirmed unresectable locally advanced, recurrent or metastatic PDAC.
17. Subjects must not be suitable for radical treatment methods, such as radical chemoradiotherapy and/or surgery; for subjects who have previously received (neo) adjuvant chemotherapy/adjuvant (radiotherapy) chemotherapy/radical radiochemotherapy, the time from the last treatment to disease recurrence is > 6 months.
18. Patients who have not received any systemic treatment (including chemotherapy, targeted therapy, tumor immunotherapy, etc.) in the locally advanced or recurrent stage.

Note:
* CLDN18.2-positive: defined as Claudin18.2 immunohistochemical membrane staining intensity ≥ 2+ in ≥ 40% of tumor cells. Previous test results, test results from the study site, and test results from the central laboratory are acceptable. The proportion of CLDN18.2 expression can be dynamically adjusted by the sponsor based on newly generated data during the study.

A subject will not be enrolled in this study if any of the following criteria are met:
1. Participating in another interventional clinical study, except for observational (non-interventional) clinical studies or in the survival follow-up stage of interventional studies.
2. Treatment with a strong inhibitor of cytochrome P450 3A4 (CYP3A4) within 2 weeks or 5 half-lives (whichever is longer) prior to the first dose of study drug.
3. Received the last anti-tumor treatment within 4 weeks or 5 half-lives of the anti-tumor drug (whichever is shorter) before the first dose of the study drug.
4. Received therapeutic or palliative radiotherapy within 2 weeks prior to the first dose of the study drug.
5. Received biliary stent implantation within 7 days prior to the first dose of the study drug.
6. Plan to receive other anti-tumor treatments during the drug therapy of this study [palliative radiotherapy for the purpose of alleviating symptoms (such as pain) without affecting the efficacy evaluation is allowed].
7. Vaccination with any live vaccine within 4 weeks prior to the first dose of study drug or planned for the vaccination during the study.
8. Major surgery (craniotomy, thoracotomy, or laparotomy, or other defined by the investigator, excluding needle biopsy) or unhealed wounds, ulcers, or fractures within 4 weeks prior to the first dose of the study drug; or plan to require major surgery during the study period. Local surgical treatment of isolated lesions with palliative intent is acceptable.
9. Previous toxicity that has not recovered to Grade 0 or 1 as per NCI CTCAE v5.0 (excluding alopecia, asthenia, pigmentation, and other conditions that are judged by the investigator to pose no safety risk) prior to the first dose of the study drug.
10. History of gastrointestinal perforation and/or fistula within 6 months prior to the first dose of study drug, which has not been cured by surgical treatment.
11. Presence of pyloric obstruction and/or persistent recurrent vomiting (≥ 3 times in 24 hours).
12. After implantation of stents in the digestive tract [the muscular conduit from the oral cavity to the anal canal, including the oral cavity, pharynx, esophagus, stomach, small intestine (duodenum, jejunum, ileum), large intestine (cecum, appendix, colon, rectum), and anal canal, etc.] or tracheal cavity.
13. Symptomatic central nervous system metastases. Subjects with asymptomatic brain metastases (i.e., no neurological symptoms, no need for glucocorticoid treatment, and brain metastases ≤ 1.5 cm) or stable symptoms of brain metastases after treatment must meet all of the following criteria to be eligible for participation in this study: no metastases to midbrain, pons, cerebellum, meninges, medulla oblongata, or spinal cord; stable clinical status for at least 4 weeks, no new or enlarged brain metastases confirmed by clinical evidence, and discontinuation of corticosteroids and anticonvulsants for at least 2 weeks prior to the first dose of study drug. Note: The central nervous system is not a target lesion.
14. Bone metastasis with risk of paraplegia.
15. Interstitial lung disease requiring steroid therapy, or history of interstitial lung disease, non-infectious pneumonitis, severely impaired lung function, or uncontrolled lung disease, such as pulmonary fibrosis, severe radiation pneumonitis, acute lung injury, etc., or suspected of having the above diseases during the screening period.
16. Presence of uncontrolled diseases, such as:
   a. Presence of poorly controlled infection requiring systemic antiinfectives (antibiotics, antivirals, or antifungals) within one week prior to the first dose of study drug.
   b. Human immunodeficiency virus (HIV) infection (positive for HIV 1/2 antibody).
   c. Acute or chronic active hepatitis B (defined as hepatitis B surface antigen (HBsAg) and/or hepatitis B core antibody (HBcAb) positive and hepatitis B virus DNA copy number ≥ 10 ⁴ copies/mL or ≥ 2000 IU/mL, or acute or chronic active hepatitis C [hepatitis C virus antibody (HCVAb) positive, HCV RNA > 10³ copies/mL]. Subjects who are less than the above criteria after nucleotide antiviral therapy and subjects who are HCV antibody positive but RNA test negative are allowed to be enrolled.
   d. Active COVID-19 infection.
   e. Active tuberculosis, receiving anti-tuberculosis treatment or received anti-tuberculosis treatment within 1 year prior to the first dose of study drug.
   f. Active syphilis or latent syphilis requiring treatment.
   g. Symptomatic congestive heart failure (New York Heart Association Class NYHA Class II-IV), symptomatic or poorly controlled arrhythmia, QTc interval >480 ms, or personal or family history of congenital long/short QT syndrome.
   h. Poorly controlled hypertension (systolic blood pressure ≥ 160 mmHg or diastolic blood pressure ≥ 100 mmHg) by standardized treatment.
   i. Tumor invades or compresses surrounding important structures (such as large blood vessels, trachea, etc.) or poses a risk of gastrointestinal/respiratory fistulae.
   j. Symptomatic pleural, ascites, or pericardial effusion requiring intervention (e.g., drainage).
   k. Subjects with esophageal or gastric varices requiring immediate intervention (e.g., banding or sclerotherapy), or who are considered by the investigator or in consultation with a gastroenterologist or hepatologist to be at high risk for bleeding, have evidence of portal hypertension (including splenomegaly on imaging), or have a history of variceal bleeding must have had an endoscopic evaluation within 3 months of first dose of study drug.
   l. Any life-threatening bleeding event or Grade 3 or 4 gastrointestinal/variceal bleeding event requiring transfusion, endoscopy, or surgery within 3 months prior to the first dose of study drug.
   m. Any arterial thromboembolic event within 6 months prior to the first dose of study drug, including myocardial infarction, unstable angina, cerebrovascular accident, transient ischemic attack, etc.
   n. History of new or uncontrolled stable deep vein thrombosis, pulmonary embolism, or any other serious venous thromboembolism within 3 months prior to the first dose of the study drug (implantable venous ports or catheter-derived thrombosis, or superficial venous thrombosis are not considered as "serious" venous thromboembolism).
   o. Hepatic encephalopathy, hepatorenal syndrome, or cirrhosis with Child-Pugh Class B or more severe.
   p. With a risk of intestinal obstruction or perforation (including but not limited to a history of acute diverticulitis or abdominal abscess) or a history of the following diseases: inflammatory bowel disease or extensive bowel resection (partial colectomy or extensive small bowel resection accompanied with chronic diarrhea), Crohn's disease, and ulcerative colitis.
   q. Significant malnutrition (if intravenous nutritional supplement is required, weight loss of 5% within 1 month or weight loss of more than 15% within 3 months, or food intake decreased by 1/2 or more within 1 week before signing the informed consent form). Except for those whose malnutrition has been corrected for more than 4 weeks prior to the first dose of study drug.
   r. Other acute or chronic diseases or laboratory abnormalities that may lead to the following results: increase the risk associated with study participation or study drug administration, or interfere with the interpretation of study results, and list the subject as ineligible for participation in this study according to the investigator's judgment.
   s. A neurological, psychiatric, or social condition that: impacts compliance with study requirements, significantly increases the risk of AEs, or impacts the participant's ability to provide a written ICF.
17. History of other primary malignant tumors, except for:
   - Radically cured malignant tumors with no known active disease for ≥ 2 years before enrollment and with an extremely low risk of recurrence;
   - adequately treated non-melanoma skin cancer or lentigo maligna without evidence of disease recurrence;
   - adequately treated carcinoma in situ without evidence of disease recurrence.
18. Known history of immunodeficiency.
19. History of allogeneic organ transplantation and allogeneic hematopoietic stem cell transplantation.
20. Cohort A3, Cohort B:
   Chronic use of systemic hormonal or immunosuppressive medication that has not been reduced to physiologic doses or discontinued 4 weeks prior to first dose, excluding: Intranasal inhaled topical steroid therapy or local steroid injection (e.g., intra-articular injection); systemic corticosteroids therapy at doses not exceeding 10 mg/day of prednisone or its equivalent physiological dose; glucocorticoids as prophylactics for allergic reactions (e.g., premedication for CT), and prophylactics for vomiting specified in the protocol. Requiring long-term systemic hormonal or any other immunosuppressive drug therapy, excluding inhaled hormone therapy.

Subjects with active autoimmune disease or inflammatory disease [including inflammatory bowel disease (such as ulcerative colitis, Crohn's disease, etc.), celiac disease, systemic lupus erythematosus, Sarcoidosis syndrome or Wegener syndrome (granulomatosis with polyangiitis), Graves' disease, rheumatoid arthritis, hypophysitis, uveitis, etc.], or a history of this disease within the past 2 years. Subjects with vitiligo, psoriasis, alopecia, or Graves' disease not requiring systemic treatment within 2 years, hypothyroidism only requiring thyroid hormone replacement therapy, and type 1 diabetes only requiring insulin replacement therapy may be enrolled. Subjects with only positive autoimmune antibodies should be confirmed for the presence of autoimmune diseases according to the investigator's judgment.
21. Prior treatment with topoisomerase inhibitor-based antibody-drug conjugates.
22. For subjects receiving drug therapy, previous history of allergy to the corresponding drug or formulation.
23. For subjects receiving drug therapy, there are contraindications to the corresponding drugs.
24. For subjects receiving drug therapy, there is a medical history of corresponding drug-related adverse reactions leading to permanent discontinuation.
25. Pregnant or lactating female subjects.
26. Other conditions that are not eligible to participate in this study considered by the researchers.

### 5. Study design

This study is a phase II study to evaluate the safety, tolerability, pharmacokinetics, and efficacy of the Claudin18.2-targeted antibody-drug conjugate of the present invention in a combination therapy for subjects with advanced malignant solid tumors, including Part 1 (safety introduction stage) and Part 2 (POC test stage).

In Part 1, 3-6 subjects are planned to be enrolled in each cohort to receive the combination therapy with the Claudin18.2-targeted antibody-drug conjugate of the present invention. The preset safe introduction dose of the Claudin18.2-targeted antibody-drug conjugate of the present invention is 6 mg/kg Q3W, and the mode of administration is intravenous infusion (IV) Q3W. The DLT observation period is within 21 days (3 weeks) after the first dose, and during the DLT observation period, only 1 dose of the Claudin18.2-targeted antibody-drug conjugate of the present invention is administered. If a DLT event is observed in ≥ 1/3 of the subjects during the DLT observation period, 3-6 subjects are allowed to be enrolled and treated with the Claudin18.2-targeted antibody-drug conjugate of the present invention at 4.5 mg/kg Q3W; if the 4.5 mg/kg dose group is intolerant, 3-6 subjects are allowed to be enrolled and treated with the Claudin18.2-targeted antibody-drug conjugate of the present invention at 3 mg/kg Q3W; if it is still not tolerated, the combination therapy exploration is terminated. If the Claudin18.2-targeted antibody-drug conjugate of the present invention is well tolerated at 6 mg/kg Q3W, the sponsor will decide whether to safely introduce it at 8 mg/kg Q3W according to the above principles based on previous study data.

The sponsor is allowed to adjust the safe introduction dose of the Claudin18.2-targeted antibody-drug conjugate of the present invention in each cohort based on the results of previous studies. After the safety introduction stage was completed and the dose and safety of the CLAUDIN18.2-targeted antibody-drug conjugate combination therapy were determined, Part 2 would be initiated.

The study cohorts and Part 2 (POC test stage) are designed as follows. Whether each cohort is initiated and the timing of initiation will be determined by the sponsor:
Cohort A: subjects with CLDN18.2-positive advanced gastric/gastroesophageal junction adenocarcinoma (G/GEJ AC) who have failed or are intolerant to first-line standard treatment will be divided into 3 groups, with about 49 subjects planned to be enrolled in each group (including subjects in the same dose group during the safety introduction period):
   A1: receiving treatment with the Claudin18.2-targeted antibody-drug conjugate of the present invention + ramucirumab 8 mg/kg d1, d15, Q4W (d represents day, the same applies below).
   A2: receiving treatment with the Claudin18.2-targeted antibody-drug conjugate of the present invention + bevacizumab 15 mg/kg Q3W.
   A3: receiving treatment with the Claudin18.2-targeted antibody-drug conjugate of the present invention + sintilimab 200 mg IV Q3W (if the safety introduction of cohort B2 has been completed before the start of this cohort, it does not need to be repeated).
Cohort B: HER2-negative and CLDN18.2-positive advanced G/GEJ AC subjects who have not received systemic treatment. After the safety introductionperiod, subjects are planned to be stratified based on an equal ratio and randomized enrolled into the following groups, with about 40 subjects in each group. Stratification factors include CPS (≥ 5 vs. < 5) and cancer site (stomach vs. gastroesophageal junction):
   B1: receiving treatment with the Claudin18.2-targeted antibody-drug conjugate of the present invention + sintilimab 200 mg IV Q3W + oxaliplatin 130 mg/m² IV Q3W. Each subject will receive up to 6 cycles of oxaliplatin treatment.
   B2: receiving treatment with the Claudin18.2-targeted antibody-drug conjugate of the present invention + sintilimab 200 mg IV Q3W.
   B3: receiving treatment with sintilimab 200 mg IV Q3W + capecitabine 1000 mg/m2 BID PO × 14d Q3W + oxaliplatin 130 mg/m² IV Q3W (oxaliplatin for up to 6 cycles).
   B4: (if B1 is well tolerated) receiving treatment with the Claudin18.2-targeted antibody-drug conjugate of the present invention + sintilimab 200 mg IV Q3W + capecitabine 1000 mg/m² BID PO × 14d Q3W + oxaliplatin 130 mg/m² IV Q3W (oxaliplatin for up to 6 cycles). The sponsor will decide whether to include this combination therapy group based on the preliminary data.
Cohort C: subjects with CLDN18.2-positive pancreatic ductal adenocarcinoma (PDAC) who failed or were intolerant to previous treatment with gemcitabine-based regimens, with about 44 subjects planned to be enrolled (including subjects in the same dose group during the safety introduction period) to receive treatment with CLAUDIN18.2-targeted antibody-drug conjugate + tegafur, gimeracil and oteracil potassium 40-60 mg, twice daily, d1-d14, Q3W.
Cohort D: subjects with CLDN18.2-positive advanced PDAC who have not received systemic treatment. After the safety introduction period, about 80 subjects are planned to be enrolled and randomized in a 1:1 ratio to D1/D2. Stratification factors include liver metastasis (yes vs. no) and primary lesion resection (yes vs. no):
   Cohort D1: receiving treatment with the Claudin18.2-targeted antibody-drug conjugate of the present invention + gemcitabine 1000 mg/m² Q3W + albumin-bound paclitaxel 125 mg/m² d1, d8, Q3W.
   Cohort D2: receiving treatment with gemcitabine 1000 mg/m² Q3W + albumin-bound paclitaxel 100 mg/m², d1, d8, Q3W.

The sponsor is allowed to adjust the requirements for the CLDN18.2 expression level of subjects enrolled in the above cohorts according to the study results of other trials of the Claudin18.2-targeted antibody-drug conjugate of the present invention. Subjects will continue the treatment until progressive disease, intolerable toxicity, withdrawal of informed consent, loss to follow-up, death, or other reasons for discontinuation of the study treatment (whichever occurs first). After study treatment discontinuation, subjects will undergo safety follow-up and survival follow-up. During the study, the investigator will perform imaging assessments for subjects according to RECIST v1.1.

The dose and treatment regimen of the study drugs are shown in Table 4 below.

**Table 4**

| **Drug Product** | **Dose/Amount** | **Frequency of Administration** | **Usage Method** | **Treatment Course/Treatment Cycle** | **Uses** |
|---|---|---|---|---|---|
| **Cohort A** | | | | | |
| **Drug Product** | **Dose/Amount** | **Frequency of Administration** | **Usage Method** | **Treatment Course/Treatment Cycle** | **Uses** |
| **A1** | | | | | |
| CLAUDIN18.2-targeted antibody-drug conjugate of the present invention | The starting dose in Part 1 is 6 mg/kg; the dose in Part 2 will be determined based on the results of Part 1 | Q3W | Intravenous infusion | Every 21 days as a cycle | Test group |
| | | | | Administration on Day 1 | |
| Ramucirumab | 8 mg/kg | Q4W | Intravenous infusion | Every 28 days as a cycle | |
| | | | | Administration on Day 1 and Day 15 | |

| **A2** | | | | | |
|---|---|---|---|---|---|
| CLAUDIN18.2-targeted antibody-drug conjugate of the present invention | The starting dose in Part 1 is 6 mg/kg; the dose in Part 2 will be determined based on the results of Part 1 | Q3W | Intravenous infusion | Every 21 days as a cycle | Test group |
| | | | | Administration on Day 1 | |
| Bevacizumab | 15 mg/kg | Q3W | Intravenous infusion | Every 21 days as a cycle | |
| | | | | Administration on Day 1 | |

| **A3** | | | | | |
|---|---|---|---|---|---|
| CLAUDIN18.2-targeted antibody-drug conjugate of the present invention | The starting dose in Part 1 is 6 mg/kg; the dose in Part 2 will be determined based on the results of Part 1 | Q3W | Intravenous infusion | Every 21 days as a cycle | Test group |
| | | | | Administration on Day 1 | |
| Sintilimab | 200 mg | Q3W | Intravenous infusion | Every 21 days as a cycle | |
| | | | | Administration on Day 1 | |

| **Cohort B** | | | | | |
|---|---|---|---|---|---|
| **B1** | | | | | |
| **Drug Product** | **Dose/Amount** | **Frequency of Administration** | **Usage Method** | **Treatment Course/Treatment Cycle** | **Uses** |
| CLAUDIN18.2-targeted antibody-drug conjugate of the present invention | The starting dose in Part 1 is 6 mg/kg; the dose in Part 2 will be determined based on the results of Part 1 | Q3W | Intravenous infusion | Every 21 days as a cycle | Test group |
| | | | | Administration on Day 1 | |
| Sintilimab | 200 mg | Q3W | Intravenous infusion | Every 21 days as a cycle | |
| | | | | Administration on Day 1 | |
| Oxaliplatin * | 130 mg/m² | Q3W | Intravenous infusion | Every 21 days as a cycle | |
| | | | | Administration on Day 1 | |
| *Each subject will receive up to 6 cycles of oxaliplatin treatment. | | | | | |

| **B2** | | | | | |
|---|---|---|---|---|---|
| CLAUDIN18.2-targeted antibody-drug conjugate of the present invention | The starting dose in Part 1 is 6 mg/kg; the dose in Part 2 will be determined based on the results of Part 1 | Q3W | Intravenous infusion | Every 21 days as a cycle | Test group |
| | | | | Administration on Day 1 | |
| Sintilimab | 200 mg | Q3W | Intravenous infusion | Every 21 days as a cycle | |
| | | | | Administration on Day 1 | |

| **B3** | | | | | |
|---|---|---|---|---|---|
| Sintilimab | 200 mg | Q3W | Intravenous infusion | Every 21 days as a cycle | Test group |
| | | | | Administration on Day 1 | |
| Oxaliplatin * | 130 mg/m² | Q3W | Intravenous infusion | Every 21 days as a cycle | |
| | | | | Administration on Day 1 | |

| **Drug Product** | **Dose/Amount** | **Frequency of Administration** | **Usage Method** | **Treatment Course/Treatment Cycle** | **Uses** |
|---|---|---|---|---|---|
| Capecitabine | 1000 mg/m² | BID | Oral administration | Every 21 days as a cycle | |
| | | | | Administration on Days 1-14 | |
| *Each subject will receive up to 6 cycles of oxaliplatin treatment | | | | | |

| **B4** | | | | | |
|---|---|---|---|---|---|
| CLAUDIN18.2-targeted antibody-drug conjugate of the present invention | The starting dose in Part 1 is 6 mg/kg; the dose in Part 2 will be determined based on the results of Part 1 | Q3W | Intravenous infusion | Every 21 days as a cycle | Test group |
| | | | | Administration on Day 1 | |
| Sintilimab | 200 mg | Q3W | Intravenous infusion | Every 21 days as a cycle | |
| | | | | Administration on Day 1 | |
| Oxaliplatin * | 130 mg/m² | Q3W | Intravenous infusion | Every 21 days as a cycle | |
| | | | | Administration on Day 1 | |
| Capecitabine | 1000 mg/m² | BID | Oral administration | Every 21 days as a cycle | |
| | | | | Administration on Days 1-14 | |
| *Each subject will receive up to 6 cycles of oxaliplatin treatment. | | | | | |

| **Cohort C** | | | | | |
|---|---|---|---|---|---|
| CLAUDIN18.2-targeted antibody-drug conjugate of the present invention | The starting dose in Part 1 is 6 mg/kg; the dose in Part 2 will be determined based on the results of Part 1 | Q3W | Intravenous infusion | Every 21 days as a cycle | Test group |
| | | | | Administration on Day 1 | |
| Tegafur, gimeracil and oteracil potassium | 40mg-60mg | BID | Oral administration | Every 21 days as a cycle | |
| | | | | Administration on Days 1-14 | |

| **Drug Product** | **Dose/Amount** | **Frequency of Administration** | **Usage Method** | **Treatment Course/Treatment Cycle** | **Uses** |
|---|---|---|---|---|---|
| **Cohort D** | | | | | |
| **D1** | | | | | |
| CLAUDIN18.2-targeted antibody-drug conjugate of the present invention | The starting dose in Part 1 is 6 mg/kg; the dose in Part 2 will be determined based on the results of Part 1 | Q3W | Intravenous infusion | Every 21 days as a cycle | Test group |
| | | | | Administration on Day 1 | |
| Gemcitabine | 1000 mg/m² | Q3W | Intravenous infusion | Every 21 days as a cycle | |
| | | | | Administration on Day 1 and Day 8 | |
| Albumin-bound paclitaxel | 125 mg/ m² | Q3W | Intravenous infusion | Every 21 days as a cycle | |
| | | | | Administration on Day 1 and Day 8 | |

| **D2** | | | | | |
|---|---|---|---|---|---|
| Gemcitabine | 1000 mg/m² | Q3W | Intravenous infusion | Every 21 days as a cycle | Test group |
| | | | | Administration on Day 1 and Day 8 | |
| Albumin-bound paclitaxel | 125 mg/ m² | Q3W | Intravenous infusion | Every 21 days as a cycle | |
| | | | | Administration on Day 1 and Day 8 | |

### 6. Evaluation indicators

### Safety assessments

Incidence, correlation with the investigational drug, and severity of all TEAEs, AESIs, and SAEs.

Changes in vital signs, ECG, physical examination, and laboratory test results before, during, and after treatment with the investigational drug.

Immunogenicity: All subjects will be tested for ADA, and ADA-positive serum samples will be tested for neutralizing antibody (NAb) (if necessary).

### PK assessment

PK characteristics of the drug after single and multiple doses in humans to determine key PK parameters, including but not limited to: AUC, Tmax, Ctrough, Cmax, CL, V, and t1/2.

### Efficacy assessments

The investigator will evaluate the tumor efficacy according to RECIST v1.1, and the evaluation indicators include: objective response rate (ORR), duration of response (DoR), disease control rate (DCR), time to response (TTR), and progression-free survival (PFS).
Overall survival (OS).
Biomarker assessment
CLDN18.2 expression level in tumor tissues before treatment.

### 7. Statistical methods

### Sample size

The sample size of each cohort in Part 1 is not based on statistical hypothesis testing. For each combination therapy regimen, 3-6 subjects are planned to be enrolled at each dose level, and a total of about 21-84 subjects are planned.

Part 2 is to evaluate the efficacy and safety of the CLAUDIN18.2-targeted antibody-drug conjugate of the present invention in combination therapy for subjects with advanced malignant solid tumors, comprising four cohorts, in which about 431 subjects will be enrolled: Cohort A comprises 3 cohorts, with 49 subjects in each group (including subjects in the same dose group of Part 1), totaling 147 subjects; cohort B comprises up to four treatment groups, with 40 subjects in each cohort, totaling 160 subjects; cohort C comprises 44 subjects (including subjects in the same dose group of Part 1); cohort D comprises two treatment groups, with 40 subjects in each group, totaling 80 subjects. The sample size for each cohort is calculated as follows:
Cohort A includes subjects with CLDN18.2-positive advanced G/GEJ AC who failed or were intolerant to first-line standard treatment. According to the safety results of Part 1, three test groups were planned to be conducted: the CLAUDIN18.2-targeted antibody-drug conjugate of the present invention is combined with sintilimab, ramucirumab, and bevacizumab, respectively. Assuming that the ORR of each group of combination drugs is 45% and the dropout rate is 20%. A sample size of 49 subjects will have 70% power to ensure that the lower limit of the 90% confidence interval is > 26.5%.
Cohort B includes subjects with HER2-negative and CLDN18.2-positive advanced G/GEJ AC who have not received systemic treatment. It is planned to conduct an equal-ratio randomized controlled trial: B1, B2 and B4 (if included) are the test group, and B3 is the control group. B1: three-drug combination (CLAUDIN18.2-targeted antibody-drug conjugate of the present invention + sintilimab + oxaliplatin). B2: two-drug combination (CLAUDIN18.2-targeted antibody-drug conjugate of the present invention + sintilimab). B3: Standard control treatment (sintilimab + capecitabine + oxaliplatin). B4: four-drug combination (CLAUDIN18.2-targeted antibody-drug conjugate of the present invention + sintilimab + capecitabine + oxaliplatin, if included). A total of 160 subjects (if group B4 is included) or 120 subjects (if group B4 is not included) are planned to be enrolled with 40 subjects in each group. Assuming that the ORR of each treatment group is 78% and the ORR of the control group is 58%, the one-sided significance level for inter-group comparison is 0.1, and the dropout rate is 10%. 80 subjects have about 70% power to detect that the ORR of group B1/B2/B4 is higher than that of group B3.
Cohort C includes CLDN18.2-positive PDAC subjects who failed or were intolerant to previous treatment with the gemcitabine-based regimen, and received the two-drug combination therapy of the CLAUDIN18.2-targeted antibody-drug conjugate of the present invention + tegafur, gimeracil and oteracil potassium. Assuming that the ORR of the combination drug is 35% and the dropout rate is 20%. The sample size of 44 subjects will have 70% power to ensure that the lower limit of the 90% confidence interval is > 17%.
Cohort D includes subjects with CLDN18.2-positive advanced pancreatic ductal adenocarcinoma (PDAC) who had not previously received systemic treatment. A 1:1 randomized controlled trial is planned: the CLAUDIN18.2-targeted antibody-drug conjugate of the present invention + gemcitabine + albumin-bound paclitaxel (test group), and gemcitabine + albumin-bound paclitaxel (control group). A total of 80 subjects were planned to be enrolled with 40 subjects in each group. Assuming that the ORR of the test group is 43% and the ORR of the control group is 23%, the one-sided significance level for inter-group comparison is 0.1, and the dropout rate is 10%. 80 subjects will have about 70% power to detect that the ORR of the test group is higher than that of the control group.

### Statistical Methods

Unless otherwise specified, subjects in Part 1 and Part 2 (non-randomized cohorts A and C) with the same tumor type/dose/combination therapy regimen will undergo statistical analysis after data are combined, and DLT data in Part 1 will be analyzed separately by tumor type /dose level/combination therapy regimen.

All safety, PK, immunogenicity, and efficacy data will be summarized using descriptive statistics. Continuous variables will be summarized using descriptive statistics (including number of subjects, mean, standard deviation, median, minimum, and maximum, and PK parameters also include geometric mean and geometric coefficient of variation). Discrete variables will be summarized using frequency and percentage.

### Anti-tumor efficacy indicators include:

- Best overall efficacy [(complete response (CR], partial response (PR), stable disease (SD), or progressive disease (PD)]
- ORR (CR+PR) and DCR (CR+PR+SD)
- DoR and TTR
- PFS
- OS

If the data permit, ORR and DCR and their confidence intervals will be provided, and the proportion of subjects whose best response is CR, PR, SD, or PD will also be calculated. For randomized cohorts in Part 2, the chi-square test will be used to compare the inter-group differences in ORR and DCR, and the inter-group difference in ORR rate and its confidence interval will be estimated; the Kaplan-Meier method will be used to calculate the median time of DoR, TTR, PFS, and OS and their confidence intervals, as well as the rate at specified time points (if applicable, e.g., at month 6, month 12, and every 6 months thereafter) and their confidence intervals. For randomized cohorts in Part 2, the log-rank test will also be used to compare the inter-group differences, and the Cox model will be used to calculate the HR and its confidence interval.

Safety data: All AEs will be coded and classified according to Medical Dictionary for Regulatory Activities (MedDRA), and the incidence and severity of various adverse events will be summarized. Descriptive statistical methods will be used to analyze the observed values and changes from baseline of vital signs, laboratory tests, and 12-lead ECG.

### Immunogenicity data: The positive rates of anti-drug antibodies and neutralizing antibodies will be summarized

PK data: descriptive statistical analysis of PK measurement data and its parameters, including but not limited to AUC, Cmax, CL, V, and t1/2.

Biomarker data: The expression level of CLDN18.2 in tumor tissues before treatment was analyzed using descriptive statistics, and the relationship between the expression level of CLDN18.2 before treatment and efficacy was explored.

### 8. Experimental results

1) In this experiment, Balb/c mice were inoculated with EMT6-hClaudin18.2 cells to determine the anti-tumor effect of IEX019-02 (i.e., a CLAUDIN18.2-targeted antibody-drug conjugate) in combination with an anti-PD-1 antibody

### Balb/c mice:

Female Balb/c background mice were purchased from Beijing Vital River Laboratory Animal Technology Co., Ltd., SPF grade. The study was initiated after the mice were acclimated for 3 days upon arrival.

### Cells:

EMT6-hClaudin18.2 cells (produced by Innovent Biologics (Suzhou) Co., Ltd.) were routinely subcultured in strict accordance with the instructions for use for subsequent in vivo experiments. The cells were collected by centrifugation, resuspended in sterile PBS, and adjusted to a cell density of 15 × 10⁶ cells/mL. On day 0, 0.2 mL of the cell suspension was subcutaneously inoculated into the right abdominal region of Balb/c mice to establish EMT6-hClaudin18.2 tumor-bearing mouse models.

### Administration:

7 days after the tumor cell inoculation, the tumor volume in each mouse was measured with a vernier caliper. Mice with a tumor volume of about 97-137 mm³ were selected and evenly grouped according to the tumor volume (7 mice per group). The dosages and routes of administration are shown in Table 5. hIgG (purchased from EQUITECH-BIO) was used as a negative control, and αPD-1 was a murine PD-1 monoclonal antibody (see PCT/CN2016/094122 for the sequence). The CLAUDIN18.2-targeted antibody-drug conjugate was administered once a week for a total of three times, i.e., on days 7, 14 and 21 after inoculation, and αPD-1 was administered twice a week for a total of 5 times, i.e., on days 7, 10, 14, 17 and 21 after inoculation. The tumor volume and body weight of the mice were monitored twice a week. The body weight and tumor volume were measured before each administration, and the relative tumor growth inhibition rate (TGI%) was calculated on day 24 after inoculation. The calculation formula is as follows: TGI% = 100% * (control group tumor volume - treatment group tumor volume)/(control group tumor volume - control group tumor volume before administration). Tumor volume measurement: The maximum length of long axis (L) and maximum length of wide axis (W) of tumors were measured with a vernier caliper, and tumor volume was calculated using the following formula: V = L ×W2/2. The body weight was measured using an electronic balance.

**Table 5. Experimental design**

| Group | Dose of administration | Number of doses | Mode of administration |
|---|---|---|---|
| hIgG | 15 mg/kg | Q3-4Dx5 | Intraperitoneal injection |
| CLAUDIN18.2-targeted antibody-drug conjugate | 10 mg/kg | QWx3 | Intraperitoneal injection |
| αPD-1 | 5 mg/kg | Q3-4Dx5 | Intraperitoneal injection |
| CLAUDIN18.2-targeted antibody-drug conjugate + αPD-1 | 10 mg/kg+5 mg/kg | QWx3+Q3-4Dx5 | Intraperitoneal injection |

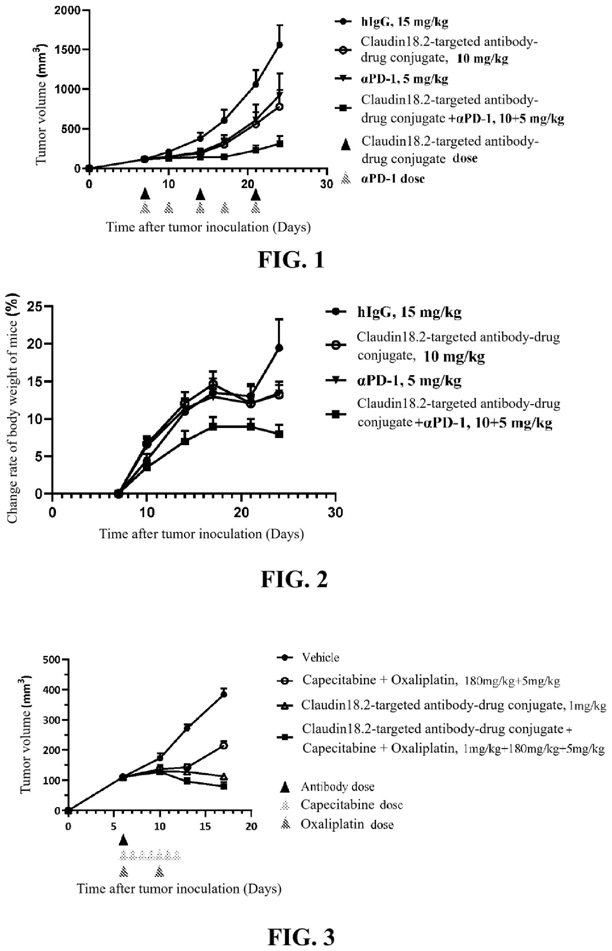

The tumor growth inhibition rate results are shown in Table 6 and FIG. 1: the tumor growth inhibition rate of the CLAUDIN18.2-targeted antibody-drug conjugate group was 54%, the tumor growth inhibition rate of the αPD-1 group was 44%, the tumor growth inhibition rate of the CLAUDIN18.2-targeted antibody-drug conjugate + αPD-1 group was 86%, and the tumor growth inhibition effect of the CLAUDIN18.2-targeted antibody-drug conjugate + αPD-1 group was stronger than that of the CLAUDIN18.2-targeted antibody-drug conjugate monotherapy group and the αPD-1 monotherapy group. Meanwhile, the body weight of the mice was monitored, and the results are shown in FIG. 2. There was no significant difference in the body weight of the mice. Therefore, compared to the CLAUDIN18.2-targeted antibody-drug conjugate monotherapy and αPD-1 monotherapy alone, the CLAUDIN18.2-targeted antibody-drug conjugate in combination with αPD-1 has a more significant inhibitory effect on tumors.

**Table 6. Tumor growth inhibition on day 24**

| Group | Tumor volume (mm³) | TGI (%) |
|---|---|---|
| hIgG | 1559 | N/A |
| CLAUDIN18.2-targeted antibody-drug conjugate | 776 | 54 |
| αPD-1 | 918 | 44 |
| CLAUDIN18.2-targeted antibody-drug conjugate + αPD-1 | 311 | 86 |

### 2) Anti-tumor efficacy of IEX019-02 (i.e., a CLAUDIN18.2-targeted antibody-drug conjugate) in combination with capecitabine and oxaliplatin in HCT15-Claudin 18.2 tumor-bearing CB17-SCID mice

In this experiment, CB17-SCID mice were inoculated with HCT15-Claudin 18.2 cells to determine the anti-tumor effect of IEX019-02 (i.e., a CLAUDIN18.2-targeted antibody-drug conjugate) in combination with capecitabine and oxaliplatin.

### CB17-SCID mice:

Female CB17-SCID mice were purchased from Beijing Vital River Laboratory Animal Technology Co., Ltd., SPF grade, quality inspection unit: Beijing Vital River Laboratory Animal Technology Co., Ltd., certificate No. 110011231104500063. The study was initiated after the mice were acclimated for 3 days upon arrival.

### Cells:

HCT15-Claudin 18.2 cells (produced by Innovent Biologics (Suzhou) Co., Ltd.) were routinely subcultured in strict accordance with the instructions for use for subsequent in vivo experiments. The cells were collected by centrifugation, resuspended in sterile PBS, and adjusted to a cell density of 10 × 10⁶ cells/mL. On day 0, 0.2 mL of the cell suspension was subcutaneously inoculated into the right abdominal region of the CB17-SCID mice to establish HCT15-Claudin 18.2 tumor-bearing mouse models.

### Administration:

Six days after the tumor cell inoculation, the tumor volume of each mouse was measured, and mice with the tumor volume within a certain range (89.91 mm³ -138.27 mm³ ) were selected and divided into 4 groups according to the tumor volume: Vehicle group, capecitabine + oxaliplatin 180 mg/kg + 5 mg/kg group, CLAUDIN18.2-targeted antibody-drug conjugate 1 mg/kg group, and CLAUDIN18.2-targeted antibody-drug conjugate + capecitabine + oxaliplatin 1 mg/kg + 180 mg/kg + 5 mg/kg group, with 7 mice in each group. The CLAUDIN18.2-targeted antibody-drug conjugate was administered by intravenous injection at a single dose; capecitabine was administered by intragastric injection at a frequency of once daily for 7 consecutive days; oxaliplatin was administered by intraperitoneal injection at a frequency of once every 4 days for 2 consecutive doses. Tumor volume was monitored for 17 days. At the end of the experiment, the relative tumor growth inhibition rate of each group of mice was calculated. The tumor volumes of the mice was measured on days 6, 10, 13, and 17 after tumor inoculation. The tumors were measured twice a week and the mice were weighed at the time of tumor measurement and at the time of intragastric injection. On day 17 after inoculation, the relative tumor growth inhibition rate (TGI%) was calculated by the following formula: TGI% = 100% * (control group tumor volume - treatment group tumor volume)/(control group tumor volume - control group tumor volume before administration). Tumor volume measurement: The maximum length of long axis (L) and maximum length of wide axis (W) of tumors were measured with a vernier caliper, and tumor volume was calculated using the following formula: V = L ×W2/2. The body weight was measured using an electronic balance. The experimental design is shown in Table 7.

**Table 7. Experimental design**

| Group | Dose of administration | Number of doses | Mode of administration |
|---|---|---|---|
| Excipient | 10 mg/kg | One dose | Intravenous injection |
| Capecitabine + Oxaliplatin, 180 + 5 mg/kg | Capecitabine 180 mg/kg | QDx7 | Intragastric |
| | Oxaliplatin 5 mg/kg | Q4Dx2 | Intraperitoneal injection |
| CLAUDIN18.2-targeted antibody-drug conjugate, 1 mg/kg | 1 mg/kg | Single dose | Intravenous injection |
| CLAUDIN18.2-targeted antibody-drug conjugate + Capecitabine + Oxaliplatin, 1 + 180 + 5 mg/kg | CLAUDIN18.2-targeted | Single dose | Intravenous injection |
| | antibody-drug conjugate 1 mg/kg, Capecitabine 180 mg/kg | QDx7 | Intragastric |
| | | Q4Dx2 | Intraperitoneal injection |
| | Oxaliplatin 5 mg/kg | | |

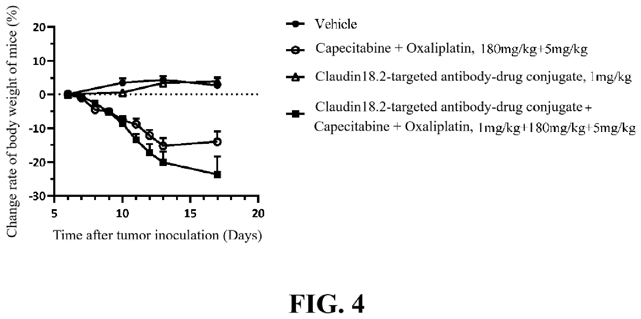

The tumor growth inhibition rate results are shown in Table 8 and FIG. 3: the tumor growth inhibition rate of the capecitabine + oxaliplatin group was 62%, the tumor growth inhibition rate of the CLAUDIN18.2-targeted antibody-drug conjugate group was 99%, the tumor growth inhibition rate of the CLAUDIN18.2-targeted antibody-drug conjugate + capecitabine + oxaliplatin group was 111%, and the tumor growth inhibition effect of the CLAUDIN18.2-targeted antibody-drug conjugate + capecitabine + oxaliplatin group was stronger than that of the capecitabine + oxaliplatin group and the CLAUDIN18.2-targeted antibody-drug conjugate monotherapy group. Therefore, the CLAUDIN18.2-targeted antibody-drug conjugate in combination with capecitabine and oxaliplatin shows a certain synergistic effect compared to the CLAUDIN18.2-targeted antibody-drug conjugate alone and the combination of capecitabine and oxaliplatin, with TGI% increased by 12% and 49%, respectively, compared to the latter two groups. Meanwhile, we monitored the body weight of the mice, and the results are shown in FIG. 4. There was no significant difference in the body weight of the mice in the CLAUDIN18.2-targeted antibody-drug conjugate group, and the body weight of the mice in the capecitabine + oxaliplatin group and the CLAUDIN18.2-targeted antibody-drug conjugate + capecitabine + oxaliplatin group was seriously reduced, and the combined administration of 180 mg/kg capecitabine and 5 mg/kg oxaliplatin could cause the body weight loss of the mice.

**Table 8. Tumor growth inhibition rate on day 17**

| Group | Tumor volume (mm³) | TGI (%) |
|---|---|---|
| Excipient | 384 | N/A |
| Capecitabine + Oxaliplatin, 180 + 5 mg/kg | 214 | 62 |
| CLAUDIN18.2-targeted antibody-drug conjugate, 1 mg/kg | 113 | 99 |
| CLAUDIN18.2-targeted antibody-drug conjugate + capecitabine + oxaliplatin, 1 + 180 + 5 mg/kg | 80 | 111 |

The Claudin18.2-targeted antibody-drug conjugate of the present invention, as an ADC with bystander effect, also has a significant tumor inhibitory effect in a mouse tumor-bearing model with low CLDN18.2 expression. In preclinical pharmacodynamic studies, it showed a certain synergistic effect in combination with an antibody drug for treating cancer and/or a chemotherapy drug, and showed a significant inhibitory effect on tumor volume. Therefore, the Claudin18.2-targeted antibody-drug conjugate of the present invention, in combination with an antibody drug for treating cancer and/or a chemotherapy drug, has good efficacy in treating subjects with various tumor types and different CLDN18.2 expression levels.

Treatment of CLDN18.2-positive advanced gastric/gastroesophageal junction adenocarcinoma that has failed or is intolerant to first-line standard treatment, with the Claudin18.2-targeted antibody-drug conjugate in combination with sintilimab, ramucirumab, or bevacizumab, respectively, can reach an ORR of more than 45%, improve PFS and OS, and have good tolerability.

Treatment of HER2-negative and CLDN18.2-positive gastric/gastroesophageal junction adenocarcinoma that have not received systemic treatment, with the Claudin18.2-targeted antibody-drug conjugate in combination with sintilimab and/or capecitabine and/or oxaliplatin, can achieve an ORR of more than 78%, improve PFS and OS, and have good tolerability.

Treatment of CLDN18.2-positive pancreatic ductal adenocarcinoma that failed or is intolerant to the previous gemcitabine-based treatment regimens, with the Claudin18.2-targeted antibody-drug conjugate in combination with tegafur, gimeracil and oteracil potassium, can achieve an ORR of more than 35%, improve PFS and OS, and have good tolerability.

Treatment of CLDN18.2-positive pancreatic ductal adenocarcinoma that has not been systematically treated, with the Claudin18.2-targeted antibody-drug conjugate in combination with gemcitabine + albumin-bound paclitaxel, can achieve an ORR of more than 43%, improve PFS and OS, and have good tolerability.

### Sequence information

| SEQ ID NO | | HB37A6 (HCDR1 is defined using AbM rules, HCDR2, HCDR3, and LCDR1, 2 and 3 are defined using Kabat rules) |
|---|---|---|
| 1 | HCDR1 | GFTFSSYVMS |
| 2 | HCDR2 | TISHSGGSTYYADSVKG |
| 3 | HCDR3 | DAPYYDILTGYRY |
| 4 | Heavy chain variable region (VH) | EVQLLDSGGGLVQPGGSLRLSCAASGFTFSSYVMSWVRQAPGKGLNWVSTIS HSGGSTYYADSVKGRFTISRDNSKNTLYLQMNSLRAEDTAVYYCAIDAPYYD ILTGYRYWGQGTLVTVSS |
| 5 | Heavy chain constant region (HC) | |
| 6 | LCDR1 | RASQSISSWLA |
| 7 | LCDR2 | KASSLES |
| 8 | LCDR3 | QQYNSYSYT |
| 9 | Light chain variable region (VL) | DIQMTQSPSTLSASVGDRVTITCRASQSISSWLAWYQQKPGKAPKLLIYKASSL ESGVPSRFSGSGSGTEFTLTISSLQPDDFATYYCQQYNSYSYTFGQGTKLEIK |
| 10 | Light chain constant region (LC) | RTVAAPSVFIFPPSDEQLKSGTASVVCLLNNFYPREAKVQWKVDNALQSGNS QESVTEQDSKDSTYSLSSTLTLSKADYEKIIKVYACEVTIIQGLSSPVTKSFNRG EC |
| 11 | Heavy chain | EVQLLDSGGGLVQPGGSLRLSCAASGFTFSSYVMSWVRQAPGKGLNWVSTIS HSGGSTYYADSVKGRFTISRDNSKNTLYLQMNSLRAEDTAVYYCAIDAPYYD ILTGYRYWGQGTLVTVSSASTKGPSVFPLAPSSKSTSGGTAALGCLVKDYFPE PVTVSWNSGALTSGVHTFPAVLQSSGLYSLSSVVTVPSSSLGTQTYICNVNHK PSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTVDKS RWQQGNVFSCSVMHEALHNHYTQKSLSLSPGK |
| 12 | Light chain | DIQMTQSPSTLSASVGDRVTITCRASQSISSWLAWYQQKPGKAPKLLIYKASSL ESGVPSRFSGSGSGTEFTLTISSLQPDDFATYYCQQYNSYSYTFGQGTKLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRGE C |

The exemplary embodiments of the present invention have been described above, and it should be understood by those skilled in the art that these disclosures are merely exemplary and that various other substitutions, adaptations, and modifications can be made within the scope of the present invention. Accordingly, the present invention is not limited to the specific embodiments set forth herein.

## Claims

1. A method for treating cancer with a Claudin18.2-targeted antibody-drug conjugate, comprising administering to an individual in need thereof an effective amount of the Claudin18.2-targeted antibody-drug conjugate, and an antibody drug for treating cancer and/or a chemotherapy drug;
wherein the Claudin18.2-targeted antibody-drug conjugate is selected from an antibody-drug conjugate of formula IB: 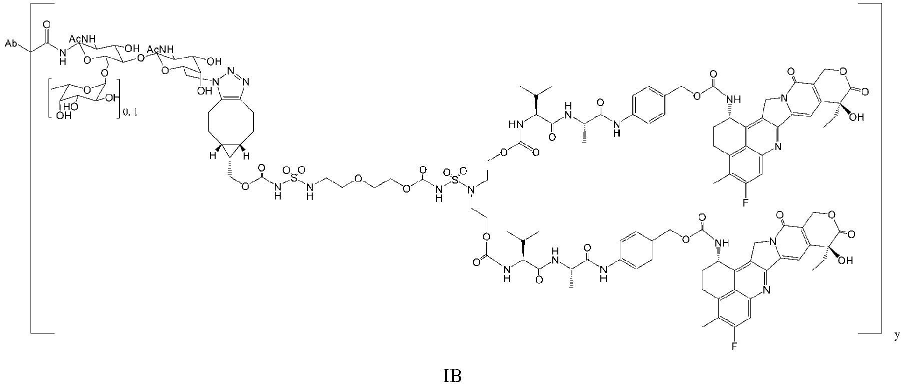
wherein Ab is a Claudin18.2-targeted antibody or an antigen-binding fragment thereof; and
wherein y is an integer of 1 or 2.

2. A method for treating cancer with a Claudin18.2-targeted antibody-drug conjugate, comprising administering to an individual in need thereof an effective amount of the Claudin18.2-targeted antibody-drug conjugate, and an antibody drug for treating cancer and/or a chemotherapy drug;
wherein the Claudin18.2-targeted antibody-drug conjugate is selected from: 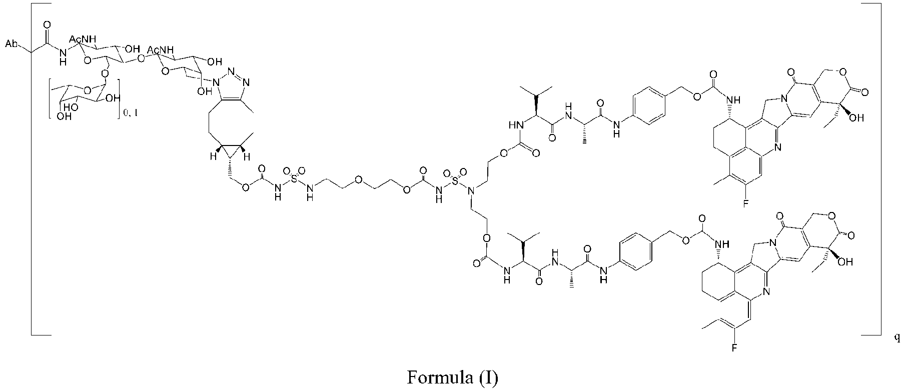
wherein Ab is a Claudin18.2-targeted antibody or an antigen-binding fragment thereof;
wherein q represents an average DAR; q is 2-11, for example, a value of 2 to 5, preferably a value of 3 to 5, and more preferably a value of 3 to 4.

3. The method according to claim 1 or 2, wherein the Claudin18.2-targeted antibody or the antigen-binding fragment thereof comprises HCDR1, HCDR2 and HCDR3 having amino acid sequences set forth in SEQ ID NOs: 1, 2 and 3, respectively, and LCDR1, LCDR2 and LCDR3 having amino acid sequences set forth in SEQ ID NOs: 6, 7 and 8, respectively; or the Claudin18.2-targeted antibody or the antigen-binding fragment thereof comprises HCDR1-3 contained in a heavy chain variable region set forth in SEQ ID NO: 4 and LCDR1-3 contained in a light chain variable region set forth in SEQ ID NO: 9; preferably, the heavy chain variable region CDRs and the light chain variable region CDRs of the Claudin18.2-targeted antibody or the antigen-binding fragment thereof are determined according to kabat, AbM, Chothia, Contact and/or IMGT scheme rules.

4. The method according to any one of claims 1-3, wherein the Claudin18.2-targeted antibody or the antigen-binding fragment thereof comprises a heavy chain variable region and/or a light chain variable region, wherein the heavy chain variable region
(i) comprises or consists of an amino acid sequence set forth in SEQ ID NO: 4; or
(ii) comprises or consists of an amino acid sequence having one or more (preferably no more than 10, and more preferably no more than 5, 4, 3, 2, or 1) amino acid changes (preferably amino acid substitutions, and more preferably conservative amino acid substitutions) compared to the amino acid sequence set forth in SEQ ID NO: 4, wherein preferably, the amino acid changes do not occur in the CDR regions;
or
an amino acid sequence having at least 85%, at least 90%, at least 91%, at least 92%, at least 93%, at least 94%, at least 95%, at least 96%, at least 97%, at least 98%, or at least 99% sequence identity to the amino acid sequence of SEQ ID NO: 4; and/or
the light chain variable region
(i) comprises or consists of an amino acid sequence set forth in SEQ ID NO: 9; or
(ii) comprises or consists of an amino acid sequence having one or more (preferably no more than 10, and more preferably no more than 5, 4, 3, 2, or 1) amino acid changes (preferably amino acid substitutions, and more preferably conservative amino acid substitutions) compared to the amino acid sequence set forth in SEQ ID NO: 9, wherein preferably, the amino acid changes do not occur in the CDR regions;
or
an amino acid sequence having at least 85%, at least 90%, at least 91%, at least 92%, at least 93%, at least 94%, at least 95%, at least 96%, at least 97%, at least 98%, or at least 99% sequence identity to the amino acid sequence of SEQ ID NO: 9.

5. The method according to any one of claims 1-4, wherein the Claudin18.2-targeted antibody or the antigen-binding fragment thereof further comprises a heavy chain constant region and/or a light chain constant region.

6. The method according to claim 5, wherein the heavy chain constant region of the Claudin18.2-targeted antibody or the antigen-binding fragment thereof is an IgG antibody.

7. The method according to claim 6, wherein the heavy chain constant region of the Claudin18.2-targeted antibody or the antigen-binding fragment thereof is selected from IgG1 or IgG2 or IgG3 or IgG4, preferably IgG1.

8. The method according to any one of claims 1-7, wherein the Claudin18.2-targeted antibody or the antigen-binding fragment thereof comprises a heavy chain and a light chain, wherein the heavy chain
comprises or consists of an amino acid sequence set forth in SEQ ID NO: 11; or
comprises or consists of: an amino acid sequence having one or more (preferably no more than 20 or 10, and more preferably no more than 5, 4, 3, 2, or 1) amino acid changes (preferably amino acid substitutions, and more preferably conservative amino acid substitutions) compared to the amino acid sequence set forth in SEQ ID NO: 11, or an amino acid sequence having at least 85%, at least 90%, at least 91%, at least 92%, at least 93%, at least 94%, at least 95%, at least 96%, at least 97%, at least 98%, or at least 99% sequence identity to the amino acid sequence set forth in SEQ ID NO: 11; and
the light chain
comprises or consists of an amino acid sequence set forth in SEQ ID NO: 12; or
comprises or consists of: an amino acid sequence having one or more (preferably no more than 20 or 10, and more preferably no more than 5, 4, 3, 2, or 1) amino acid changes (preferably amino acid substitutions, and more preferably conservative amino acid substitutions) compared to the amino acid sequence set forth in SEQ ID NO: 12, or an amino acid sequence having at least 85%, at least 90%, at least 91%, at least 92%, at least 93%, at least 94%, at least 95%, at least 96%, at least 97%, at least 98%, or at least 99% sequence identity to the amino acid sequence set forth in SEQ ID NO: 12.

9. The method according to any one of claims 1-8, wherein the method comprises administering to an individual in need thereof the Claudin18.2-targeted antibody-drug conjugate at 0.1 mg/kg - 100 mg/kg.

10. The method according to any one of claims 1-9, wherein the method comprises administering to an individual in need thereof the Claudin18.2-targeted antibody-drug conjugate at 0.2 mg/kg - 50 mg/kg.

11. The method according to any one of claims 1-10, wherein the method comprises administering to an individual in need thereof the Claudin18.2-targeted antibody-drug conjugate at 0.3 mg/kg - 30 mg/kg.

12. The method according to claim 11, wherein the method comprises administering to an individual in need thereof the Claudin18.2-targeted antibody-drug conjugate at 1 mg/kg, 3 mg/kg, 4.5 mg/kg, 6 mg/kg, 7.5 mg/kg, 8 mg/kg, 10 mg/kg, 12 mg/kg, 13.5 mg/kg, 15 mg/kg, 16 mg/kg, 18 mg/kg, 20 mg/kg, 22 mg/kg, 25 mg/kg, 28 mg/kg, or 30 mg/kg.

13. The method according to any one of claims 1-12, wherein the administration cycle of the Claudin18.2-targeted antibody-drug conjugate is 2 weeks/14 days (Q2W) - 5 weeks/35 days (Q5W).

14. The method according to claim 13, wherein the administration cycle of the Claudin18.2-targeted antibody-drug conjugate is 2 weeks/14 days (Q2W), 3 weeks/21 days (Q3W), 4 weeks/28 days (Q4W), or 5 weeks/35 days (Q5W), and administration is performed once on day 1 of each cycle.

15. The method according to any one of claims 1-14, wherein the antibody drug for treating cancer is selected from one or a combination of more of a PD-1 inhibitor, a recombinant anti-VEGF monoclonal antibody, and a monoclonal antibody that specifically binds to VEGFR-2.

16. The method according to claim 15, wherein the PD-1 inhibitor is selected from one or a combination of more of pembrolizumab, nivolumab, camrelizumab, sintilimab, toripalimab, and tislelizumab.

17. The method according to claim 16, wherein the method comprises administering to an individual in need thereof 200 mg of sintilimab.

18. The method according to claim 16 or 17, wherein the administration cycle of sintilimab is 3 weeks/21 days (Q3W), e.g., administration on day 1.

19. The method according to claim 15, wherein the recombinant anti-VEGF monoclonal antibody is selected from one or a combination of two of sevacizumab and bevacizumab.

20. The method according to claim 19, wherein the method comprises administering to an individual in need thereof 15 mg/kg of bevacizumab.

21. The method according to claim 20, wherein the administration cycle of bevacizumab is 3 weeks/21 days (Q3W), e.g., administration on day 1.

22. The method according to claim 15, wherein the monoclonal antibody that specifically binds to VEGFR-2 comprises ramucirumab.

23. The method according to claim 22, wherein the method comprises administering to an individual in need thereof 8 mg/kg of ramucirumab.

24. The method according to claim 23, wherein the administration cycle of ramucirumab is 4 weeks/28 days (Q4W), e.g., administration on day 1 and day 15.

25. The method according to any one of claims 1-24, wherein the chemotherapy drug is selected from one or a combination of more of oxaliplatin, capecitabine, gemcitabine, albumin-bound paclitaxel, and tegafur, gimeracil and oteracil potassium.

26. The method according to claim 25, wherein the administration dose of oxaliplatin is 130 mg/m²; the administration cycle of oxaliplatin is 3 weeks/21 days (Q3W), e.g., administration on day 1.

27. The method according to claim 25, wherein the administration dose of capecitabine is 1000 mg/m²; the administration cycle of capecitabine is twice daily.

28. The method according to claim 25, wherein the administration dose of gemcitabine is 1000 mg/m²; the administration cycle of gemcitabine is 3 weeks/21 days (Q3W), e.g., administration on day 1 and day 8.

29. The method according to claim 25, wherein the administration dose of albumin-bound paclitaxel is 125 mg/m²;
the administration cycle of albumin-bound paclitaxel is 3 weeks/21 days (Q3W), e.g., administration on day 1 and day 8.

30. The method according to claim 25, wherein the administration dose of the tegafur, gimeracil and oteracil potassium is 40-60 mg; the administration cycle of the tegafur, gimeracil and oteracil potassium is 3 weeks/21 days (Q3W), twice daily, e.g., administration on days 1-14.

31. The method according to any one of claims 1-30, wherein the cancer comprises a solid tumor, and the solid tumor comprises gastric/gastroesophageal junction adenocarcinoma and pancreatic ductal adenocarcinoma.

32. The method according to any one of claims 1-31, wherein the administration mode of the method comprises intravenous injection and oral administration.

33. The method according to any one of claims 1-31, wherein an administration regimen for the individual is selected from the group consisting of:
(1) administering the Claudin18.2-targeted antibody-drug conjugate at 4 mg/kg - 10 mg/kg (preferably 6 mg/kg), every 21 days as a cycle, administration on day 1; administering ramucirumab at 8 mg/kg, every 28 days as a cycle, administration on day 1 and day 15;
(2) administering the Claudin18.2-targeted antibody-drug conjugate at 4 mg/kg - 10 mg/kg (preferably 6 mg/kg), every 21 days as a cycle, administration on day 1; administering bevacizumab at 15 mg/kg, every 21 days as a cycle, administration on day 1;
(3) administering the Claudin18.2-targeted antibody-drug conjugate at 4 mg/kg - 10 mg/kg (preferably 6 mg/kg), every 21 days as a cycle, administration on day 1 of each cycle; administering sintilimab at 200 mg, every 21 days as a cycle, administration on day 1;
(4) administering the Claudin18.2-targeted antibody-drug conjugate at 4 mg/kg - 10 mg/kg (preferably 6 mg/kg), every 21 days as a cycle, administration on day 1 of each cycle; administering sintilimab at 200 mg, every 21 days as a cycle, administration on day 1; administering oxaliplatin at 130 mg/m², every 21 days as a cycle, administration on day 1, each subject receives up to 6 cycles of oxaliplatin;
(5) administering the Claudin18.2-targeted antibody-drug conjugate at 4 mg/kg - 10 mg/kg (preferably 6 mg/kg), every 21 days as a cycle, administration on day 1 of each cycle; administering sintilimab at 200 mg, every 21 days as a cycle, administration on day 1; administering capecitabine at 1000 mg/m² twice daily, every 21 days as a cycle, administration on days 1-14; administering oxaliplatin at 130 mg/m² , every 21 days as a cycle, administration on day 1 (oxaliplatin is administered for up to 6 cycles);
(6) administering the Claudin18.2-targeted antibody-drug conjugate at 4 mg/kg - 10 mg/kg (preferably 6 mg/kg), every 21 days as a cycle, administration on day 1 of each cycle; administering tegafur, gimeracil and oteracil potassium at 40 mg to 60 mg twice daily, every 21 days as a cycle, administration on days 1-14; and
(7) administering the Claudin18.2-targeted antibody-drug conjugate at 4 mg/kg-10 mg/kg (preferably 6 mg/kg), every 21 days as a cycle, administration on day 1 of each cycle; administering gemcitabine at 1000 mg/m², every 21 days as a cycle, administration on day 1 and day 8; administering albumin-bound paclitaxel at 125 mg/m², every 21 days as a cycle, administration on day 1 and day 8.

34. A unit formulation, wherein the unit formulation comprises an effective amount (e.g., 1 mg-10000 mg, 5 mg-9000 mg, 3 mg-8000 mg, 10 mg-6000 mg, 50 mg-4000 mg, 100 mg-2500 mg, 200 mg-1000 mg, 300 mg-900 mg, 400 mg-800 mg, 500 mg-700 mg, 180 mg-550 mg, 200 mg-540 mg, 240 mg-480 mg, 300 mg-420 mg, 360 mg-420 mg, or 360 mg-480 mg) of the Claudin18.2-targeted antibody-drug conjugate as defined in any one of claims 1-8, wherein preferably, the Claudin18.2-targeted antibody-drug conjugate is in a form suitable for injection (preferably intravenous or subcutaneous injection) or oral administration, preferably, the unit formulation further comprises instructions.

35. A single drug dose unit or kit, comprising an effective amount (e.g., 1 mg-10000 mg, 5 mg-9000 mg, 3 mg-8000 mg, 10 mg-6000 mg, 50 mg-4000 mg, 100 mg-2500 mg, 200 mg-1000 mg, 300 mg-900 mg, 400 mg-800 mg, 500 mg-700 mg, 180 mg-550 mg, 200 mg-540 mg, 240 mg-480 mg, 300 mg-420 mg, 360 mg-420 mg, or 360 mg-480 mg) of the Claudin18.2-targeted antibody-drug conjugate as defined in any one of claims 1-8, wherein preferably, the kit further comprises an effective amount (e.g., 1-10000 mg, 5-9000 mg, 3-8000 mg, 10-6000 mg, 50-4000 mg, 100-2500 mg, 200-1000 mg, 300-900 mg, 400-800 mg, 500-700 mg, 180 mg-550 mg, 200 mg-540 mg, 240 mg-480 mg, 300 mg-420 mg, 360 mg-420 mg, or 360 mg-480 mg) of an antibody drug for treating cancer and/or a chemotherapy drug; preferably, the Claudin18.2-targeted antibody-drug conjugate, the antibody drug for treating cancer and/or the chemotherapy drug are in a form suitable for injection (preferably intravenous or subcutaneous injection) or oral administration; preferably, the single drug dose unit or kit further comprises instructions.

36. Use of the Claudin18.2-targeted antibody-drug conjugate as defined in any one of claims 1-8 in the preparation of a pharmaceutical composition or kit for treating cancer, wherein the pharmaceutical composition or kit comprises an effective amount of the Claudin18.2-targeted antibody-drug conjugate and instructions; preferably, the kit further comprises a component A selected from an antibody drug for treating cancer and/or a chemotherapy drug.

37. The unit formulation according to claim 34, or the single drug dose unit or kit according to claim 35, or the use according to claim 36, wherein the instructions describes an administration regimen, for example, the administration dose of the Claudin18.2-targeted antibody-drug conjugate is 0.1 mg/kg-100 mg/kg, 0.2 mg/kg-50 mg/kg, 0.3 mg/kg-30 mg/kg, 0.3 mg/kg, 1 mg/kg, 3 mg/kg, 4.5 mg/kg, 6 mg/kg, 7.5 mg/kg, 8 mg/kg, 10 mg/kg, 11 mg/kg, 12 mg/kg, 13.5 mg/kg, 15 mg/kg, 16 mg/kg, 18 mg/kg, 20 mg/kg, 22 mg/kg, 25 mg/kg, 28 mg/kg or 30 mg/kg,
or the administration dose of the Claudin18.2-targeted antibody-drug conjugate is 1 mg-10000 mg, 5 mg-9000 mg, 3 mg-8000 mg, 10 mg-6000 mg, 50 mg-4000 mg, 100 mg-2500 mg, 200 mg-1000 mg, 300 mg-900 mg, 400 mg-800 mg, 500 mg-700 mg, 180 mg-550 mg, 200 mg-540 mg, 240 mg-480 mg, 300 mg-420 mg, 360 mg-420 mg, or 360 mg-480 mg,
the administration cycle is 2 weeks/14 days (Q2W) - 5 weeks/35 days (Q5W), preferably the administration cycle is 3 weeks/21 days (Q3W) or 4 weeks/28 days (Q4W), and the administration is performed once on day 1 of each cycle;
preferably, the administration regimen is selected from the group consisting of:
(1) administering the Claudin18.2-targeted antibody-drug conjugate at 4 mg/kg - 10 mg/kg (preferably 6 mg/kg), every 21 days as a cycle, administration on day 1; administering ramucirumab at 8 mg/kg, every 28 days as a cycle, administration on day 1 and day 15;
(2) administering the Claudin18.2-targeted antibody-drug conjugate at 4 mg/kg - 10 mg/kg (preferably 6 mg/kg), every 21 days as a cycle, administration on day 1; administering bevacizumab at 15 mg/kg, every 21 days as a cycle, administration on day 1;
(3) administering the Claudin18.2-targeted antibody-drug conjugate at 4 mg/kg - 10 mg/kg (preferably 6 mg/kg), every 21 days as a cycle, administration on day 1 of each cycle; administering sintilimab at 200 mg, every 21 days as a cycle, administration on day 1;
(4) administering the Claudin18.2-targeted antibody-drug conjugate at 4 mg/kg - 10 mg/kg (preferably 6 mg/kg), every 21 days as a cycle, administration on day 1 of each cycle; administering sintilimab at 200 mg, every 21 days as a cycle, administration on day 1; administering oxaliplatin at 130 mg/m², every 21 days as a cycle, administration on day 1, each subject receives up to 6 cycles of oxaliplatin;
(5) administering the Claudin18.2-targeted antibody-drug conjugate at 4 mg/kg - 10 mg/kg (preferably 6 mg/kg), every 21 days as a cycle, administration on day 1 of each cycle; administering sintilimab at 200 mg, every 21 days as a cycle, administration on day 1; administering capecitabine at 1000 mg/m² twice daily, every 21 days as a cycle, administration on days 1-14; administering oxaliplatin at 130 mg/m², every 21 days as a cycle, administration on day 1 (oxaliplatin is administered for up to 6 cycles);
(6) administering the Claudin18.2-targeted antibody-drug conjugate at 4 mg/kg - 10 mg/kg (preferably 6 mg/kg), every 21 days as a cycle, administration on day 1 of each cycle; administering tegafur, gimeracil and oteracil potassium at 40 mg to 60 mg twice daily, every 21 days as a cycle, administration on days 1-14; and
(7) administering the Claudin18.2-targeted antibody-drug conjugate at 4 mg/kg - 10 mg/kg (preferably 6 mg/kg), every 21 days as a cycle, administration on day 1 of each cycle; administering gemcitabine at 1000 mg/m², every 21 days as a cycle, administration on day 1 and day 8; administering albumin-bound paclitaxel at 125 mg/m², every 21 days as a cycle, administration on day 1 and day 8.

38. The unit formulation according to claim 34, or the single drug dose unit or kit according to claim 35, or the use according to claim 36, wherein the effective amount of the Claudin18.2-targeted antibody-drug conjugate is selected from the group consisting of: 1 mg-10000 mg, 5 mg-9000 mg, 3 mg-8000 mg, 10 mg-6000 mg, 50 mg-4000 mg, 100 mg-2500 mg, 200 mg-1000 mg, 300 mg-900 mg, 400 mg-800 mg, 500 mg-700 mg, 180 mg-550 mg, 200 mg-540 mg, 240 mg-480 mg, 300 mg-420 mg, 360 mg-420 mg, or 360 mg-480 mg.

39. The unit formulation according to claim 34, or the single drug dose unit or kit according to claim 35, or the use according to claim 36, wherein the Claudin18.2-targeted antibody-drug conjugate is as defined in any one of claims 1-8.

40. The single drug dose unit or kit according to claim 35, or the use according to claim 36, wherein the antibody drug for treating cancer is as defined in any one of claims 15-24, or the chemotherapy drug is as defined in any one of claims 25-30.
